(19) Europäisches Patentamt
European Patent Office
Office européen des brevets

(11) EP 1 032 584 B1

# (12) FASCICULE DE BREVET EUROPEEN

(45) Date de publication et mention de la délivrance du brevet:
**12.02.2003 Bulletin 2003/07**

(21) Numéro de dépôt: **98955663.4**

(22) Date de dépôt: **16.11.1998**

(51) Int Cl.$^7$: **C07J 41/00**, A61K 31/565, C07J 43/00, A61K 31/58, C07J 31/00

(86) Numéro de dépôt international:
**PCT/FR98/02437**

(87) Numéro de publication internationale:
**WO 99/025725 (27.05.1999 Gazette 1999/21)**

(54) **NOUVEAUX 19-NOR STERO DES SUBSTITUES EN POSITION 11BETA, PROCEDE ET INTERMEDIAIRES DE PREPARATION, APPLICATION COMME MEDICAMENTS ET COMPOSITIONS PHARMACEUTIQUES LES RENFERMANT**

NEUE 19-NOR 11-BETA-SUBSTITUIERTE STEROIDE, EIN VERFAHREN ZU IHRER HERSTELLUNG SOWIE ZWISCHENPRODUKTEN DIESES VERFAHRENS UND IHRE ANWENDUNG ALS MEDIKAMENTEN

NOVEL 19-NOR STEROIDS SUBSTITUTED IN POSITION 11BETA, PREPARATION METHOD AND INTERMEDIATES, APPLICATION AS MEDICINES AND PHARMACEUTICAL COMPOSITIONS CONTAINING THEM

(84) Etats contractants désignés:
**AT BE CH CY DE DK ES FI FR GB GR IE IT LI LU NL PT SE**
Etats d'extension désignés:
**AL LT LV MK RO SI**

(30) Priorité: **17.11.1997 FR 9714357**

(43) Date de publication de la demande:
**06.09.2000 Bulletin 2000/36**

(73) Titulaire: **Aventis Pharma S.A.**
**92160 Antony (FR)**

(72) Inventeur: **NIQUE, François**
**F-94170 Le Perreux sur Marne (FR)**

(56) Documents cités:
**EP-A- 0 384 842**
**FR-A- 2 640 977**
**US-A- 4 978 657**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 032 584 B1

## Description

La présente invention concerne des composés 19-nor stéroïdes, substitués en position 11β, leurs procédé et intermédiaires de préparation, leur application comme médicament et les compositions pharmaceutiques les renfermant.

L'ostéoporose est une pathologie qui se caractérise par une réduction quantitative et qualitative du tissu osseux, suffisante pour entraîner des fractures vertébrales ou périphériques, ce de façon spontanée ou à l'occasion de traumatisme minime. Bien que cette affection soit d'origine multifactorielle, c'est la ménopause qui, chez la femme, constitue le facteur prépondérant de la perte osseuse ou ostéopénie.

Cette ostéopénie se manifeste par une raréfaction et une modification de l'architecture de l'os spongieux qui a pour conséquence d'accentuer la fragilité squelettique et le risque fracturaire. La perte osseuse s'accentue fortement après la ménopause en raison de la suppression de la fonction ovarienne et atteint 3 à 5 % par an pour se ralentir après 65 ans.

Dans un but thérapeutique, la carence hormonale post ménopausique peut être compensée par une hormonothérapie substitutive où l'oestrogène joue un rôle majeur en préservant le capital osseux. Mais l'estrogénothérapie au long cours s'accompagne parfois d'effet indésirable sur l'appareil génital (hyperplasie endométriale, tumeur mammaire...), ce qui constitue un inconvénient majeur et limite son application.

Il convient donc de trouver d'autres composés que l'oestradiol ayant une activité oestrogène dissociée, à savoir une activité oestrogène au niveau osseux, tout en n'ayant pas ou peu d'activité d'hyperplasie endométriale, ni d'activité de prolifération de tumeur mammaire.

US 4,978,657 et FR-A-2640977 décrivent des dérivés stéroides substitués en position 11 beta par un radical phényle éventuellement substitué, présentant une activité hormonale ou antihormonale. Pour les composés de la présente invention, ce phényle est directement substitué par un groupement de type amino alkyle, alors que dans l'art antérieur, ce type de substituant est toujours relié au phényle par l'intermédiaire d'un hétéroatome, par exemple aminoalkyloxy.

L'invention a donc pour objet les composés de formule générale (I) :

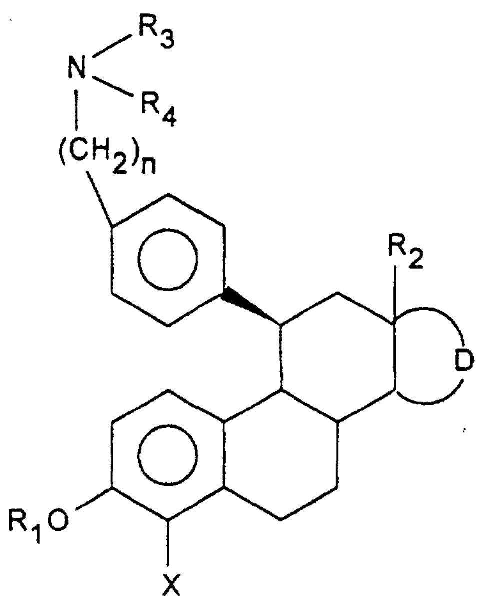

(I)

dans laquelle :

- $R_1$ représente un atome d'hydrogène, un radical $(CH_2)_m$-Ar, (CO)-Ar, $(CH_2)_m$-Alk ou (CO)-Alk,
- $R_2$ représente un radical dérivé d'un hydrocarbure, linéaire ou ramifié, saturé ou insaturé renfermant de 1 à 6 atomes de carbone
- D représente le reste d'un cycle pentagonal ou hexagonal éventuellement substitué et éventuellement porteur d'insaturation,
- X représente un atome d'halogène ou un atome d'hydrogène,
- n est égal à 3, 4 ou 5,

soit $R_3$ et $R_4$ identiques ou différents représentent un atome d'hydrogène, un groupement $(CH_2)_m$-Ar, $(CH_2)_m$-Het ou $(CH_2)_m$-Alk,
soit $R_3$ et $R_4$ forment ensemble avec l'atome d'azote auquel ils sont liés un hétérocycle mono ou polyclique, saturé ou insaturé, aromatique ou non aromatique, de 3 à 15 chaînons renfermant éventuellement de 1 à 3 hétéroatomes additionnels choisis parmi l'oxygène, le soufre et l'azote, non substitué ou substitué,
Ar représentant un groupement aryle carbocyclique renfermant de 6 à 18 atomes de carbone, Het représentant un hétérocycle aromatique ou non aromatique, saturé ou non saturé, comportant de 1 à 9 atomes de carbone et de 1 à 5 hétéroatomes choisis parmi les atomes d'oxygène, d'azote ou de soufre, Alk représentant un radical dérivé d'un hydrocarbure, non aromatique, linéaire, ramifié ou cyclique, saturé ou insaturé et comportant de 1 à 12 atomes de carbone, les radicaux Ar, Het ou Alk pouvant être substitués ou non substitués, m représente 0, 1, 2 ou 3, ainsi que leurs sels d'addition avec les bases ou les acides.

On entend par halogène : iode, brome, chlore ou fluor.

On entend par $(CH_2)_m$ les valeurs suivantes : simple liaison dans le cas où m est égal à 0, $CH_2$, $(CH_2)_2$ et $(CH_2)_3$.

Par le terme Ar représentant le groupe aryle carbocyclique renfermant de 6 à 18 atomes de carbone, on entend un dérivé d'un hydrocarbure cyclique aromatique tel que le radical phényle, naphtyle, phénanthrényle ou bien un dérivé d'un hydrocarbure bicyclique ou tricyclique condensé comportant un cycle benzénique tel que indanyle, indényle, dihydronaphtyle, tétrahydronaphtyle ou fluorényle. La jonction s'effectue au niveau du cycle benzénique. Il s'agit de préférence du phényle.

Par le terme (Het) représentant un hétérocycle aromatique ou non aromatique, saturé ou non saturé, comportant de 1 à 9 atomes de carbone et de 1 à 5 hétéroatomes choisis parmi les atomes d'oxygène, d'azote et de soufre, on désigne notamment :

- les radicaux monocyclique hétérocycliques, par exemple les radicaux thiényle, furyle, pyrannyle, pyrrolyle, imidazolyle, pyrazolyle, pyridyle, pyrazinyle, pyrimidinyle, pyridazinyle, thiazolyle, oxazolyle, furazannyle, pyrrolinyle, imidazolinyle, pyrazolinyle, thiazolinyle, triazolyle, tétrazolyle,
- les cycles condensés hétérocycliques, par exemple le benzofurannyle, le benzothiényle, le benzimidazolyle, le benzothiazolyle, le naphto[2,3-b]thiényle, le thianthrényle, l'isobenzofurannyle, le chroményle, le xanthényle, le phénoxathiinyle, l'indolizinyle, l'isoindolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle, le phénazinyle, le phénothiazinyle, le phénoxazinyle, l'indolinyle, l'isoindolinyle, l'imidazopyridyle, l'imidazopyrimidinyle ou encore les systèmes polycycliques condensés constitués de monocycliques hétérocycliques tels que définis ci-dessus comme par exemple le furo[2,3-b]pyrrole ou le thiéno[2,3-b]furanne,
- ou les hétérocycles saturés tels que pyrrolidine, pipéridine et morpholine.

Par le terme (Alk) représentant un radical dérivé d'un hydrocarbure, non aromatique, linéaire, ramifié ou cyclique, saturé ou insaturé, on désigne dans le cas des hydrocarbures acycliques les radicaux alkyles tels que méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle, les radicaux alkényles tels que vinyle, propényle, isopropényle, allyle, 2-méthylallyle, butényle ou isobutényle, ou les radicaux alkynyles tels que éthynyle, propynyle, propargyle, butynyle ou isobutynyle, et dans le cas des radicaux cycliques, les radicaux cycloalkyles, tels que cyclopropyle, cyclobutyle, cyclopentyle ou cyclohexyle.
Il s'agira de préférence des radicaux méthyle et éthyle.

Par CO-Alk on entend de préférence $COCH_3$ et COEt, par CO-Ar on entend de préférence le radical benzoyle, lorsque m est différend de zéro, $(CH_2)_m$-Ar sera de préférence le groupement benzyle.

Lorsque $R_3$ et $R_4$ forment ensemble avec l'atome d'azote auquel ils sont liés un hétérocycle, il s'agit notamment des hétérocycles mono ou bicycliques renfermant éventuellement un autre hétéroatome choisi parmi l'oxygène et l'azote tels que les hétérocycles insaturés suivants : pyrrolyle, imidazolyle, indolyle, pyridyle, pyrazinyle, pyrimidinyle, pyridazinyle, thiazolyle, oxazolyle, furazolinyle, pyrazolinyle, thiazolinyle, ou, plus particulièrement, les hétérocycles saturés suivants :

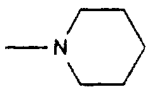 , 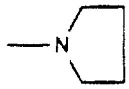 , 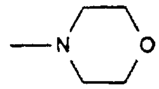 , 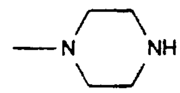 et 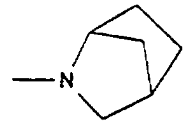

Lorsque les différents groupements Alk, Ar, Het, ainsi que le reste d'un cycle pentagonal ou hexagonal cité plus haut, sont substitués, ils peuvent l'être notamment par les radicaux suivants : halogène, à savoir fluor, chlore, brome ou iode, alkoxy tel que méthoxy, éthoxy, propyloxy, isopropyloxy, butyloxy, alkylthio tel que méthylthio, éthylthio, propylthio, isopropylthio, butylthio, amino, alkylamino tel que méthylamino ou éthylamino, dialkylamino tel que diméthylamino, diéthylamino, méthyléthylamino, chacun de ces radicaux dialkylamino étant éventuellement sous forme oxydée, aminoalkyle tel que aminométhyle ou aminoéthyle, dialkylaminoalkyle tel que diméthyl- amino méthyle ou éthyle, dialkylaminoalkyloxy tel que diméthylamino éthyloxy, hydroxyle éventuellement acylé, acyle tel que acétyle, propionyle, butyryle, benzoyle, carboxy libre, estérifié tel que alkoxy carbonyle par exemple méthoxy carbonyle ou éthoxy carbonyle, cyano, trifluorométhyle, aryle tel que phényle, aralkyle tel que benzyle, alkyle, alkényle ou alkynyle ces radicaux étant eux-mêmes éventuellement substitués par les radicaux halogène, alkyle, alkoxy, alkylthio, amino, alkylamino ou dialkylamino indiqués ci-dessus.

Bien entendu, l'expression "substitué" indique qu'un ou plusieurs substituants, identiques ou différents, peuvent être présents. A titre d'exemple, lorsque le groupement alkyl est un radical méthyl substitué par un ou plusieurs atomes d'halogènes, il peut s'agir notamment de $CH_2Cl$, $CH_2F$, $CHF_2$ et $CF_3$.
Dans le cas de (Het), les substituants peuvent être au niveau de NH ou d'un atome de carbone.

Bien entendu les valeurs de $R_1$, $R_2$, $R_3$ et $R_4$, sont indépendantes les unes des autres.

L'invention s'étend naturellement aux sels des composés de formule (I), comme par exemple les sels formés avec des acides minéraux ou organique sur l'amine. Il peut alors s'agir des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques. Lorsque les composés de formule (I) comportent une fonction acide, l'invention s'étend aux sels des métaux alcalins, alcalino terreux ou d'ammonium éventuellement substitués.

L'invention a plus particulièrement pour objet les composés de formule générale (I) telle que définie plus haut dans laquelle (D) représente le reste d'un cycle pentagonal de formule :

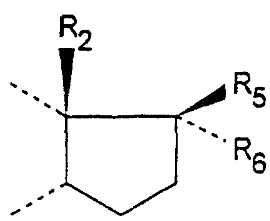

dans laquelle $R_2$ conserve la même signification que précédemment,
soit $R_5$ représente un radical OH, O-$(CH_2)_m$-Alk, O-(CO)-Alk, O-$(CH_2)_m$-Ar, O-(CO)-Ar, O-$(CH_2)_m$-Het, O-(CO)-Het et $R_6$ représente un atome d'hydrogène, un radical alkyle, alkényle ou alkynyle renfermant de 1 à 6 atomes de carbone substitué ou non substitué, m, Alk, Ar et Het étant tels que définis précédemment,
soit $R_5$ et $R_6$ forment ensemble avec l'atome de carbone qui les porte l'un des cycles suivants :

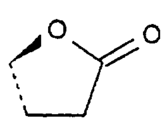 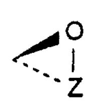 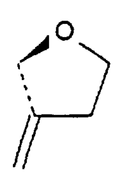

dans lequel Z représente un groupement $-(CH_2)_1-$ ou $-CH=CH-(CH_2)_{1'}$ ; l étant un entier compris entre 1 et 4 et 1' étant un entier égal à 1 ou 2,

soit $R_5$ et $R_6$ forment ensemble un groupement oxo,

ainsi que leurs sels d'addition avec les acides ou les bases.

L'invention a plus particulièrement pour objet les composés de formule (I) telle que définie précédemment répondant à la formule générale (I') :

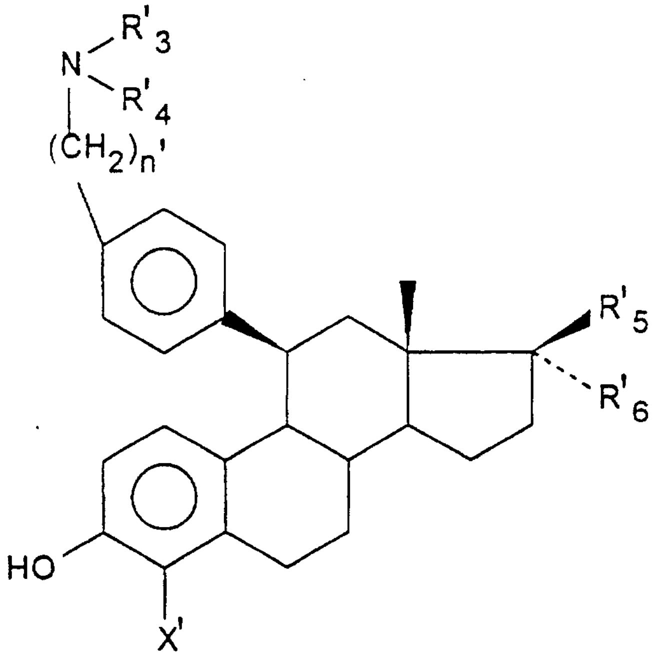

(I')

dans laquelle :

X' représente un atome de chlore, de brome ou d'hydrogène,

n' est égal à 3,

soit $R'_3$ et $R'_4$ identiques ou différents représentent un radical alkyle renfermant de 1 à 6 atomes de carbone

soit $R'_3$ et $R'_4$ forment ensemble, avec l'atome d'azote auquel ils sont liés, un reste mono ou polycyclique saturé de 3 à 15 chaînons renfermant éventuellement un hétéroatome additionnel choisi parmi l'oxygène, le soufre et l'azote,

$R'_5$ et $R'_6$ ont la même signification que $R_5$ et $R_6$,

ainsi que leurs sels d'addition avec les acides et les bases.

L'invention a plus particulièrement pour objet les composés de formule (I) telle que définie précédemment répondant à la formule générale (I') dans laquelle :

soit $R'_5$ représente un radical OH et $R'_6$ représente un atome d'hydrogène, un radical alkyle, alkényle ou alkynyle renfermant de 1 à 6 atomes de carbone, substitué ou non substitué,

soit $R'_5$ et $R'_6$ forment ensemble avec l'atome de carbone qui les porte l'un des cycles suivants :

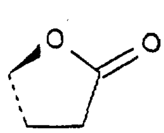
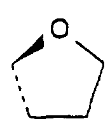
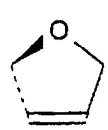
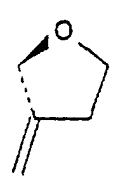

soit $R'_5$ et $R'_6$ forment ensemble un groupement oxo,

ainsi que leurs sels d'addition avec les acides ou les bases.

L'invention a plus particulièrement pour objet les composés de formule (I) répondant à la formule générale (I') telle que définie précédemment dans laquelle :

X' représente un atome de chlore ou d'hydrogène,
n' est égal à 3,
soit $R'_3$ et $R'_4$ identiques ou différents représentent un radical alkyle renfermant de 1 à 6 atomes de carbone
soit $R'_3$ et $R'_4$ forment ensemble avec l'atome d'azote les hétérocycles saturés suivants :

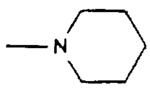 , 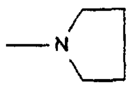 , 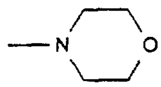 , 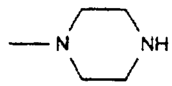 et 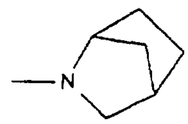

et soit $R'_5$ représente un radical OH et $R'_6$ représente un atome d'hydrogène, un radical alkyle, alkényle ou alkynyle renfermant de 1 à 6 atomes de carbone, substitué ou non substitué,
soit $R'_5$ et $R'_6$ forment ensemble avec l'atome de carbone qui les porte l'un des cycles suivants :

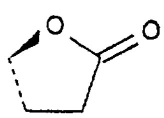 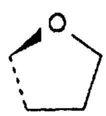 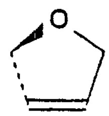 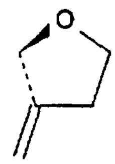

soit $R'_5$ et $R'_6$ forment ensemble un groupement oxo,

ainsi que leurs sels d'addition avec les acides ou les bases.

L'invention a plus particulièrement pour objet soit les composés de formule générale (I) telle que définie précédemment dans laquelle X = H, soit les composés de formule générale (I) telle que définie précédemment dans laquelle X = Cl ou Br, et plus particulièrement Cl.

L'invention a tout particulièrement pour objet les composés de formule (I) ainsi que leurs sels d'addition avec les acides dont les noms suivent :

3-hydroxy-11β-[4-[3-(1-pipéridinyl)propyl]phényl]-estra-1,3,5(10)-trièn-17-one,
3-hydroxy-11β-[4-[3-(1-pyrrolidinyl)propyl]phényl]-estra-1,3,5(10)-trièn-17-one,
3-hydroxy-11β-[4-[3-diméthylamino)propyl]phényl]-estra-1,3,5(10)-trièn-17-one,
4-chloro-3-hydroxy-11β-[4-[3-(1-pipéridinyl)propyl]phényl]-estra-1,3,5(10)-trièn-17-one
4-chloro-3-hydroxy-11β-[4-[3-(1-pyrrolidinyl)propyl]phényl]-estra-1,3,5(10)-trièn-17-one
4-chloro-3-hydroxy-11β-[4-[3-(diéthylamino)propyl]phényl]-estra-1,3,5(10)-trièn-17-one
11β-[4-[3-(1-pyrrolidinyl)propyl]phényl]-estra-1,3,5(10)-triène-3,17β-diol,
11β-[4-(3-diméthylamino)propyl)phényl]-estra-1,3,5(10)-triène-3,17β-diol
11β-[4-(3-(1-pipéridinyl)propyl)phényl]-estra-1,3,5(10)-triène-3,17β-diol
4-chloro-11β-[4-[3-(1-pyrrolidinyl)propyl]phényl]-estra-1,3,5(10)-triène-3,17β-diol
4-chloro-11β-[4-[3-(1-pipéridinyl)propyl]phényl]-estra-1,3,5(10)-triène-3,17β-diol
4-chloro-11β-[4-[3-(diéthylamino)propyl]phényl]-estra-1,3,5(10)-triène-3,17β-diol
17α-méthyl-11β-[4-[3-(1-pipéridinyl)propyl]phényl]-estra-1,3,5(10)-triène-3,17β-diol,
4-chloro-17α-méthyl-11β-[4-[3-(1-pipéridinyl)propyl]phényl]-estra-1,3,5(10)-triène-3,17β-diol,
11β-[4-[3-(1-pipéridinyl)propyl]phényl]-17α-(trifluorométhyl)-estra-1,3,5(10)-triène-3,17β-diol
(17R) 11β-[4-(3-diméthylamino)propyl)phényl]-spiro-(estra-1,3,5(10)-triène-17,2'(5'H)-furan)-3-ol
(17R) 4',5'-dihydro-11β-[4-(3-diméthylamino)propyl)phényl]-spiro-(estra-1,3,5(10)-triène-17,2'(3'H)-furan)-3-ol.

L'invention a également pour objet un procédé de préparation des composés de formule (I) telle que définie précédemment dans lequel l'on soumet un composé de formule (II) :

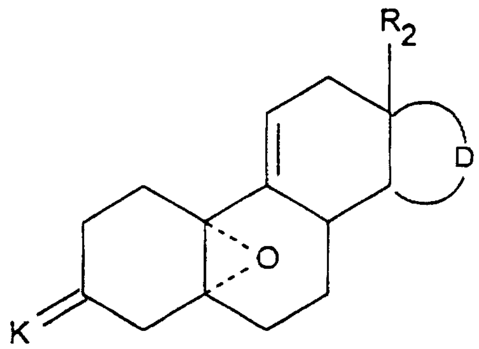

(II)

dans laquelle D et $R_2$ sont tels que définis précédemment et K représente un groupement protecteur de la fonction 3-céto, successivement aux réactions suivantes :

a) action d'un composé de formule (III) :

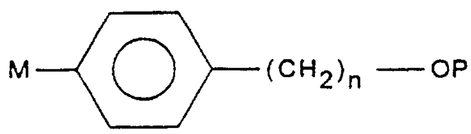

(III)

dans laquelle, M représente un dérivé métallique, P représente un groupement protecteur de l'alcool et n est un entier égal à 3, 4 ou 5, puis le cas échéant déprotection d'une ou de plusieurs des fonctions réactives protégées, afin d'obtenir un composé de formule ($III_a$) :

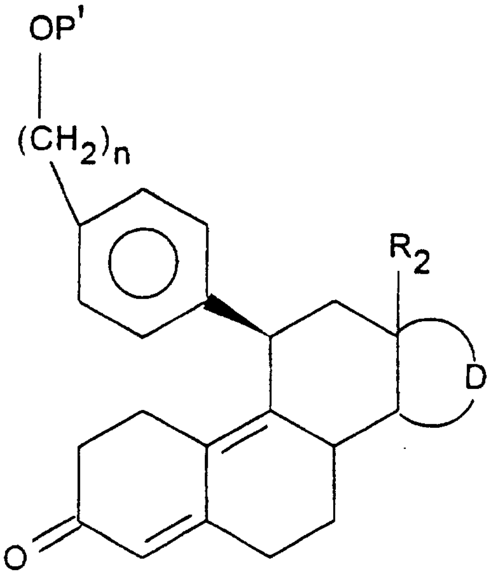

($III_a$)

P' ayant les mêmes valeurs que P ainsi que hydrogène,
b) action, le cas échéant, d'un réactif d'halogénation, afin d'obtenir un composé de formule ($III_b$) :

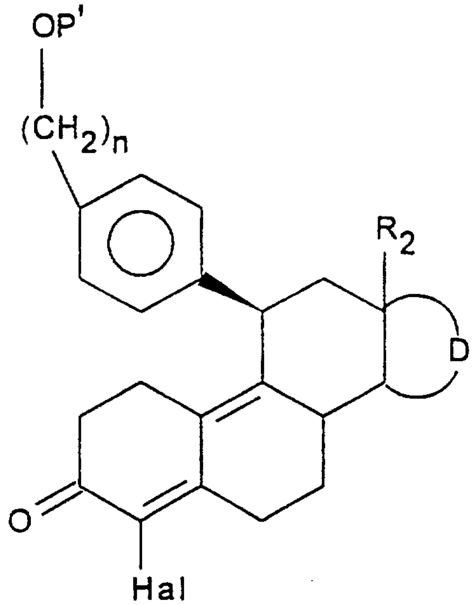

($III_b$)

Hal représentant un atome d'halogène,

c) après avoir, le cas échéant, protégé et/ou activé la fonction OH, action d'un réactif d'aromatisation du cycle (A) sur les composés de formules ($III_a$) et ($III_b$), puis action d'une base pour obtenir le composé de formule (IV) :

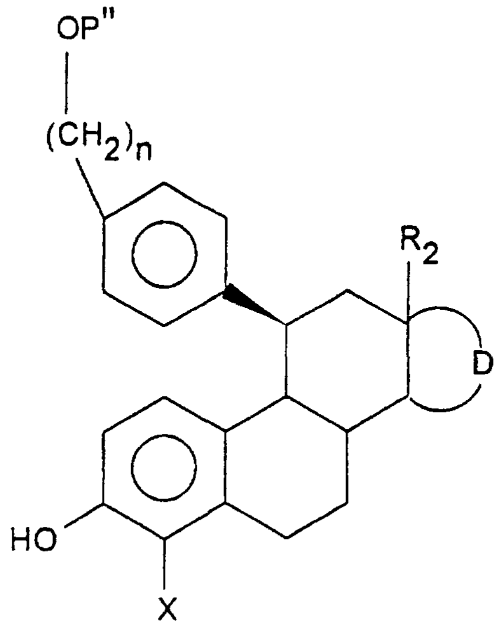

(IV)

P" ayant les mêmes valeurs que P' et pouvant représenter en outre un groupement activant,

X étant tel que défini précédemment,

d) action d'une amine de formule (V) :

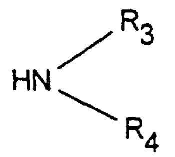

(V)

$R_3$ et $R_4$ étant tels que définis précédemment, ce composé étant éventuellement sous forme de sel, afin d'obtenir certains des composés de formule (I), les composés de formules ($III_a$), ($III_b$), (IV) et (I) étant soumis si désiré ou si nécessaire, dans un ordre approprié, à l'une ou plusieurs des réactions suivantes :

- protection/déprotection du ou des groupements OH,
- alkylation/acylation du ou des groupements OH,
- action d'un agent de réduction lorsque D représente le reste d'un cycle pentagonal tel que défini précédemment et $R_5$ et $R_6$ forment ensemble un groupement oxo,
- action d'un organométallique ou de $CF_3SiMe_3$ sur les composés de formule (IV) ou (I) avec D représentant le reste d'un cycle pentagonal tel que défini précédemment et $R_5$ et $R_6$ formant ensemble un groupement oxo,
- action d'un agent de lactonisation sur les composés de formule (IV) ou (I) avec D représentant le reste d'un cycle pentagonal tel que défini précédemment et $R_5$ et $R_6$ formant ensemble un groupement oxo,
- action d'un agent de réduction de la double liaison, lorsque D représente le reste d'un cycle pentagonal tel que défini précédemment et $R_5$ et $R_6$ forment ensemble avec le carbone qui les porte, un groupement $O-(CH_2)_{l'}-CH=CH-$,
- action d'un agent de réduction, lorsque D représente le reste d'un cycle pentagonal tel que défini précédemment, et $R_6$ est un radical alkényle ou alkynyle renfermant de 2 à 6 atomes de carbone,
- salification.

L'action d'un composé de formule (III) sur le composé de formule (II) s'effectue de préférence en présence d'un sel de cuivre tel que le chlorure de cuivre I.

L'action d'un réactif d'halogénation tel que le N-bromosuccinimide ou le N-chlorosuccinimide sur les composés de formule ($III_a$) s'effectue notamment en présence d'un solvant aprotique dipolaire tel que le diméthylformamide.

La réaction d'aromatisation suivie de la réaction de saponification (action de la base) s'effectue selon les méthodes classiques telles que décrites dans le brevet européen 0097572. On utilise de préférence un mélange d'anhydride acétique et de bromure d'acétyle comme agent d'aromatisation puis une base telle que la soude dans le méthanol comme agent de saponification.

On entend par activation de l'alcool, l'introduction notamment d'un mésylate, tosylate ou triflate qui permet de favoriser la substitution nucléophile de l'amine de formule (V) sur les composés de formule (IV). La formation du mésylate, tosylate ou triflate à partir des composés de formule ($III_a$) ou ($III_b$) avec P' représentant un hydrogène s'effectue en présence d'une base telle que la triéthylamine.

On peut également envisager la substitution de l'alcool par un atome d'halogène.

Les réactions de protection et de déprotection sont les méthodes classiques connues de l'homme du métier. Une revue assez complète se trouve dans l'ouvrage suivant : Protective groups in organic synthesis T.W greene, John Wiley & sons (1981).

Le groupement protecteur P peut représenter un radical alkyle renfermant de 1 à 4 atomes de carbone, un groupement benzyle, un groupement tétrahydropyrannyle, un groupement $R_CR_DR_ESi$, dans lequel $R_C$, $R_D$ et $R_E$ identiques ou différents, indépendamment l'un de l'autre représentent chacun un radical alkyl renfermant de 1 à 4 atomes de carbone ou un groupement phényle. Il s'agit tout particulièrement des groupements $Si(Me)_2CMe_3$ ou $-Si(Ph)_2CMe_3$ ou $-SiMe_3$.

A titre d'exemple, les réactions de déprotection de composés de formule ($III_a$) ou ($III_b$), lorsque P' est un groupement tertbutyldiphénylsilyle peuvent s'effectuer par action de fluorure de tétrabutyl ammonium en solution dans le tétrahydrofuranne.

Lorsque P' est un groupement tétrahydropyrannyle, la déprotection s'effectue en présence d'un acide aqueux dans un solvant alcoolique et de préférence par action de l'acide chlorhydrique dans le méthanol.

L'action d'un composé de formule $R_3-NH-R_4$ sur les composés de formule (IV) s'effectue dans les conditions classiques des substitutions nucléophiles, notamment en présence d'un solvant aprotique tel que le tétrahydrofuranne, OP" représente alors de préférence un groupement $O-SO_2CH_3$, $OSO_2-Ph-pMe$, $OSO_2CPh_3$. OP peut également représenter un halogène (brome ou iode de préférence).

Les réactions d'alkylation ou d'acylation du groupement OH en position 3 ou 17 sont opérées par les méthodes

classiques connues de l'homme du métier.

La réduction du 17-céto en alcool correspondant ($R_5$=OH et $R_6$=H) s'effectue selon les méthodes classiques, notamment par action d'un borohydrure alcalin tel que le borohydrure de sodium dans le méthanol ou l'éthanol ou par action de tétrahydrure d'aluminium et de lithium.

L'action d'un organométallique sur le 17-céto permet d'avoir accès aux produits de formule (I) dans laquelle D représente le reste d'un cycle pentagonal tel que défini précédemment, $R_5$ est un hydroxyle et $R_6$ représente un radical alkyle, alkényle, alkynyle éventuellement substitué.

L'organométallique dérivé d'un alkyle, alkényle ou alkynyle est choisi parmi les magnésiens de formule AlkMgHal et les lithiens de formule AlkLi dans lesquelles Alk représente un groupement alkyle, alkényle ou alkynyle renfermant au plus 8 atomes de carbone et Hal représente un atome d'halogène. Dans un mode préféré d'exécution du procédé, Hal représente un atome de chlore, de brome ou d'iode, de préférence de brome.

De préférence la réaction a lieu en présence du chlorure de cérium. Dans un mode préféré d'exécution du procédé, Hal représente un atome de chlore, de brome ou d'iode, de préférence de brome.

Pour obtenir des composés de formule (I) avec $R_5$ est un hydroxyle et $R_6$ est un groupement $CF_3$, la réaction s'effectue par action de $CF_3SiMe_3$ sur le 17-céto, suivi de l'action d'un réactif de déprotection tel que le fluorure de tétrabutylammonium.

La réaction de lactonisation à partir du 17 céto s'effectue selon la méthode de STURTZ (réf : G. STURTZ et J-J. YAOUANC, Synthesis, (1980), 289) notamment en présence de bisdiméthylamidophosphate d'allyle en présence d'un alkyllithien tel que le N-butyllithium dans le tétrahydrofuranne.

La réaction de réduction totale ou partielle lorsque $R_6$ est un radical alkényle ou alkynyle ou lorsque $R_5$ et $R_6$ forment ensemble avec le carbone qui les porte, un groupement $O-(CH_2)_{l'}-CH=CH-$, peut s'effectuer soit de manière totale par action d'hydrogène en présence d'un catalyseur tel que le palladium sur charbon ou un catalyseur au rhodium tel que le réactif de Wilkinson soit de manière partielle (alkynyle devient alkényle) par action d'un catalyseur empoisonné tel que le palladium sur sulfate de baryum empoisonné par la pyridine ou la triéthylamine.

Les réactions d'estérification et de salification sont effectuées par les méthodes courantes connues de l'homme du métier.

L'invention a plus particulièrement pour objet un procédé de préparation des composés de formule (I') telle que décrite précédemment, dans lequel on soumet un composé de formule générale (II') :

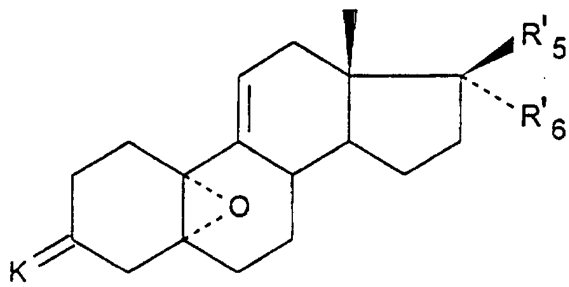

(II')

dans laquelle K, $R'_5$ et $R'_6$ sont tels que définis précédemment, ou dans laquelle $R'_5$ est un radical CN et $R'_6$ est un hydroxyle protégé, successivement aux réactions suivantes :

a) action d'un composé de formule (III') :

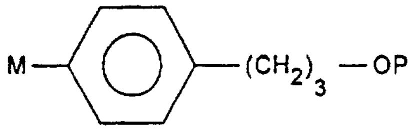

(III')

dans laquelle M et P sont tels que définis précédemment, puis déprotection d'une ou de plusieurs des fonctions réactives protégées, afin d'obtenir un composé de formule ($III'_a$) :

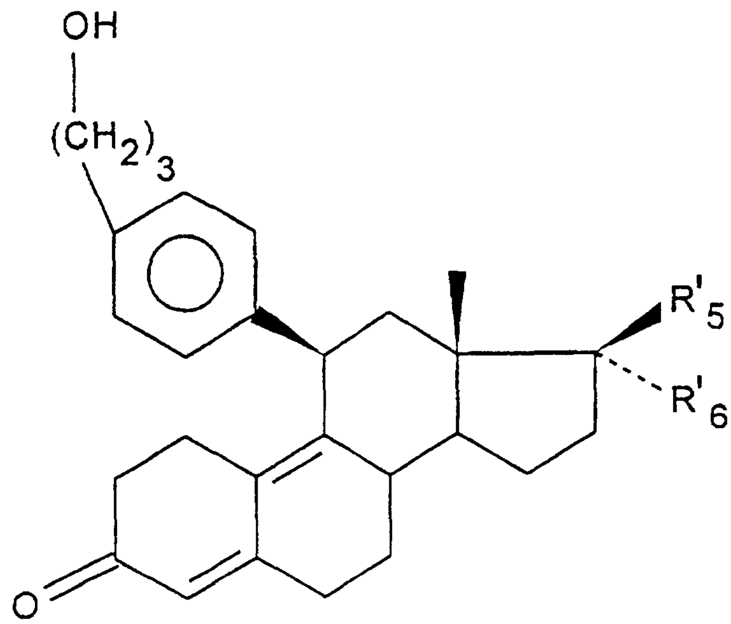

(III'a)

b) action, le cas échéant, d'un réactif d'halogénation afin d'obtenir un composé de formule ($III'_b$) :

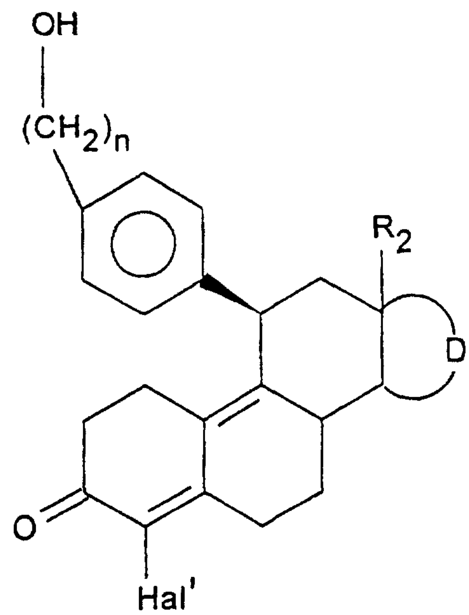

(III'b)

Hal' représentant un atome de chlore ou de brome,
c) activation de la fonction OH puis action d'un réactif d'aromatisation du cycle (A) sur les composés de formule ($III_a$) ou ($III_b$), puis action d'une base pour obtenir les composés de formule (IV') :

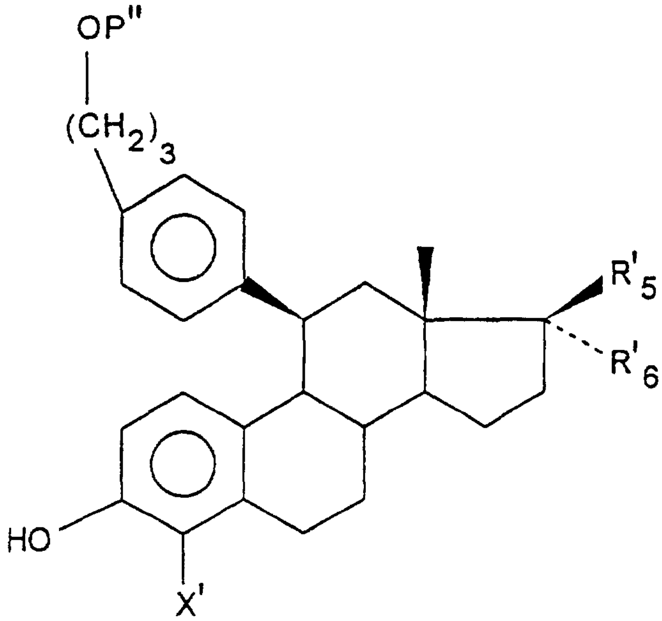

(IV')

X' et P" étant tels que définis précédemment,
d) action d'une amine de formule (V') :

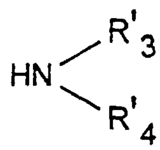

(V')

R'$_3$ et R'$_4$ écant tels que définis précédemment afin d'obtenir certains des composés de formule (I'), les composés de formules (III'$_a$), (III'$_b$), (IV') et (I') étant soumis, si désiré ou si nécessaire à l'une ou plusieurs des réactions suivantes :

- protection/déprotection du ou des groupements OH,
- alkylation/acylation du ou des groupements OH,
- action d'un agent de réduction lorsque R'$_5$ et R'$_6$ forment ensemble un groupement oxo,
- action d'un organométallique ou de $CF_3SiMe_3$ sur les composés de formule (IV') ou (I') avec R'$_5$ et R'$_6$ formant ensemble un groupement oxo,
- action d'un agent de lactonisation sur les composés de formule (IV') ou (I') avec R'$_5$ et R'$_6$ formant ensemble un groupement oxo,
- action d'un agent de réduction de la double liaison, lorsque R'$_5$ et R'$_6$ forment ensemble avec le carbone qui les porte, un groupement O-$(CH_2)_{l'}$ -CH=CH-,
- action d'un agent de réduction, lorsque R'$_6$ est un radical alkényle ou alkynyle renfermant de 2 à 6 atomes de carbone,
- salification.

Les composés de formule générale (I) ainsi que leurs sels d'addition avec les acides pharmaceutiquement acceptables possèdent notamment des activités oestrogène, anti-oestrogène et antiprolifératives.

A ce titre, les composés de formule (I) peuvent être utilisés dans le traitement des troubles liés à une hypofolliculinie, par exemple les aménorrhées, les dysménorrhées, les avortements répétés, les troubles prémenstruels, dans le traitement de certaines pathologies estrogèno-dépendantes telles que les adénomes ou les carcinomes prostatiques, les carcinomes mammaires et ses métastases ou le traitement des tumeurs bénignes du sein, en tant qu'anti-utérotrophique ainsi que dans le traitement substitutif de la ménopause ou de la périménopause.

Parmi les symptômes et les conséquences liées à la ménopause, on entend plus précisément les bouffées de chaleur, les sueurs, l'atrophie et la sécheresse vaginale, les symptômes urinaires et à long terme la diminution de la masse osseuse et l'augmentation du risque de fracture, ainsi que la perte de la protection cardio-vasculaire offerte par les oestrogènes.

En particulier, les composés de formule (I) ainsi que leurs sels d'addition avec les acides ou les bases pharmaceutiquement acceptables, peuvent ainsi être utilisés dans la prévention ou le traitement de l'ostéoporose.

Les composés de formule (I) ainsi que leurs sels d'addition avec les acides ou les bases pharmaceutiquement acceptables, peuvent également être utilisés dans la prévention ou le traitement de l'ostéoporose chez l'homme.

Ils peuvent également être utilisés dans la prévention ou le traitement des ostéoporoses secondaires (par exemple cortisoniques ou liées à une immobilisation).

Les composés de formule (I) ainsi que leurs sels d'addition avec les acides ou les bases pharmaceutiquement acceptables possèdent notamment une activité estrogénique dissociée.

Par activité estrogénique dissociée, on entend une activité estrogénique au niveau osseux tout en ne manifestant qu'une activité minimale au niveau utérin entraînant ainsi l'absence de prolifération endométriale (activité bien inférieure à celle de l'oestradiol).

Par ailleurs, les composés selon l'invention présentent les avantages suivants :

- Ils présentent une activité anti-oestrogène et/ou antiproliférative au niveau du sein. A l'opposé de l'oestradiol ils ne stimulent pas la croissance de cellules tumorales mammaires humaines et même peuvent inhiber leur croissance. Les composés selon l'invention sont donc particulièrement avantageux pour le traitement de la ménopause en ce qui concerne les femmes à risque de cancer mammaire (antécédents familiaux) qui sont donc exclues d'un traitement substitutif par l'oestradiol.

  Ils peuvent être également utilisables dans le traitement des cancers mammaires.
- Ils entraînent un abaissement du taux de cholestérol sérique à un niveau au moins équivalent à celui induit par l'estradiol. Ils renforcent ainsi la protection cardiovasculaire.
- Enfin les composés selon l'invention ne présentant aucune activité oestrogène au niveau utérin, ne nécessitent pas d'être administrés en association avec un composé progestomimétique.

L'invention a donc pour objet les composés de formule (I) ainsi que leurs sels d'addition avec les acides ou les bases pharmaceutiquement acceptables, à titre de médicaments.

L'invention a plus particulièrement pour objet les composés de formule (I) ainsi que leurs sels d'addition avec les acides ou les bases pharmaceutiquement acceptables, à titre de médicaments destinés à la prévention ou au traitement de l'ostéoporose.

L'invention s'étend aux compositions pharmaceutiques renfermant comme principe actif au moins l'un des médicaments tels que définis ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, d'anneaux intravaginal, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie utile varie en fonction de l'affection à traiter et de la voie d'administration ; elle peut varier par exemple de 1 à 1000 mg par jour chez l'adulte par voie orale.

Les composés de formule générale (II) ou (II') sont des composés connus et décrits dans le Brevet Européen 0057115.

Les composés de formule (III) sont connus ou sont aisément accessible à l'homme du métier à partir des halogénures aromatiques correspondant. Les amines de formule (V) sont également connus ou aisément accessibles à l'homme du métier.

L'invention a également pour objet, à titre de produits intermédiaires, les composés de formule ($III_a$), ($III_b$), ($III'_a$), ($III'_b$), (IV) ou (IV').

Les exemples ci-dessous illustrent l'invention sans toutefois la limiter.

Solvants décrits dans les exemples : AcOEt (acétate d'éthyle), TEA (triéthylamine), $CH_2Cl_2$ (dichlorométhane), $CHCl_3$ (chloroforme), MeOH (méthanol), $NH_4OH$ (hydroxyde d'ammonium), iPrOH (alcool isopropylique).

**Préparation 1 : 11β-[4(3-hydroxypropyl)phényl]-estra-4,9-diène-3,17-dione.**

Stade A : Alkylation

3-(4-bromophényl)-2-propynol

A une solution sous gaz inerte de 55,2 g de 4-bromo iodo benzène à 97 % dans 230 ml de DMF, on ajoute 56 ml de TEA, 12,2 ml d'alcool propargylique, 1 g d'iodure de cuivre et 1,1 g de $PdCl_2(PPh_3)_2$ tout en maintenant la température à 47°C. Après agitation pendant 3 heures 15 minutes à température ambiante, on verse dans l'eau, extrait, lave sèche et évapore sous pression réduite jusqu'à obtention de 48,3 g de produit brut que l'on purifie par chromatographie sur silice en éluant avec le mélange $CH_2Cl_2$/AcOEt 95/5. On obtient 36,37 g de produit pur attendu. (F=80°C)
Rf ($CH_2Cl_2$/AcOEt 95/5) : 0,32

| IR ($CHCl_3$) | |
|---|---|
| OH | 3609 $cm^{-1}$ |
| C≡C | 2240 $cm^{-1}$ |
| Aromatique | 1585 et 1486 $cm^{-1}$ |

Stade B : Réduction

3-(4-bromophényl)-propanol.

A une solution sous gaz inerte de 36,4 g de 3-(4-bromophényl)-2-propynol (stade A) dans 200 ml d'éthanol à 5 % de toluène, on ajoute 200 ml de toluène, 7,9 g de réactif de Wilkinson et hydrogène à 1900 mbar pendant 5 heures. On évapore sous pression réduite jusqu'à obtention de 45,9 g de produit brut que l'on purifie par chromatographie sur silice en éluant avec le mélange $CH_2Cl_2$/AcOEt 95/5. On obtient 30,1 g de produit attendu.
Rf ($CH_2Cl_2$/AcOEt 95/5) : 0,28

| IR ($CHCl_3$) | |
|---|---|
| OH | 3626 $cm^{-1}$ |
| Aromatique | 1592 et 1489 $cm^{-1}$ |

Stade C : Protection de l'alcool

(1,1-diméthyléthyl)diméthyl[[3-(4-bromophényl)propyl]oxy] silane

A une solution sous gaz inerte de 30,1 g de 3-(4-bromophényl)-propanol (stade B) dans 300 ml de $CH_2Cl_2$ on ajoute 11,4 g d'imidazole et 23 g de chlorure de diméthylterbutylsilyle. Après agitation pendant 45 minutes à température ambiante, on lave à l'eau, sèche et évapore sous pression réduite jusqu'à obtention de 47,46 g de produit brut que l'on purifie (après avoir ajouté 1,5 g d'un essai identique) par rectification. On obtient 44,88 g de produit pur attendu.
Rf ($CH_2Cl_2$/AcOEt 95/5) : 0,8

| IR ($CHCl_3$) | |
|---|---|
| OSi | 1527 $cm^{-1}$ et 836 $cm^{-1}$ |
| Aromatique | 1590 $cm^{-1}$ (f) et 1489 $cm^{-1}$ |

Stade D : Introduction du groupement aryl en position 11 du stéroïde

11β-[4-(3-hydroxypropyl)phényl]-estra-4,9-diène-3,17-dione

**Préparation du magnésien**

A 2,67 g de magnésium (tournures) dans 5 ml de THF sous atmosphère inerte et à température ambiante, on ajoute en 50 minutes au reflux après amorçage au 1,2-dibromoéthane, une solution de 32,9 g de (1,1-diméthyléthyl)diméthyl[[3-(4-bromophényl)propyl]oxy]silane (stade C) dans 100 ml de THF et maintient 5 heures au reflux. (Titre par iodométrie : 0,86M)

**Ouverture de l'époxyde**

Au mélange constitué de 120 ml du magnésien, obtenu à l'étape précédente et de 600 mg de chlorure de cuivre, on ajoute sous atmosphère inerte à 0-5°C une solution de 17,18 g de 5α,10α-époxy-3,3-[1,2-éthanediylbis (oxy)]-17α-[(triméthylsilyl)oxy]-estr-9(11)-ène-17α-carbonitrile (préparé selon le procédé décrit dans J.C. Gasc et L. Nedelec Tetrahedron Letters (1971), 2005) dans 100 ml de THF, agite 45 minutes à cette température puis verse dans une solution de chlorure d'ammonium, extrait, lave et évapore sous pression réduite jusqu'à obtention de 43,5 g de produit brut.

**Hydrolyse acide**

A une solution de 43,5 g du produit obtenu à l'étape précédente dans 300 ml de méthanol, sous atmosphère inerte et à température ambiante, on ajoute 60 ml d'acide chlorhydrique 6M et agite 1 heure à température ambiante. Après distillation du méthanol, on ajoute de l'acétate d'éthyle, lave, sèche et évapore sous pression réduite jusqu'à obtention de 30 g de produit brut (F = 254°C).

**Clivage de la cyanhydrine**

A une solution de 30 g du produit obtenu à l'étape précédente dans 200 ml de méthanol, sous atmosphère inerte et à température ambiante, on ajoute 8 ml de lessive de soude et agite 1 heure 15 à température ambiante. Après distillation du méthanol, on ajoute de l'acétate d'éthyle, lave, sèche et évapore sous pression réduite jusqu'à obtention de 27,9 g de produit brut que l'on purifie d'abord par chromatographie en éluant avec le mélange $CH_2Cl_2$/MeOH 95/5. On obtient 13 g de produit attendu (F=192°C, Rf ($CH_2Cl_2$/MeOH 95/5) : 0,28) puis par dissolution dans un mélange de 70 ml de $CH_2Cl_2$ et de 70 ml d'éther isopropylique que l'on concentre jusqu'à cristallisation.

On obtient 11,92 g de produit pur attendu. (Rf ($CH_2Cl_2$/AcOEt 95/5) : 0,28)
F=192°C

| RMN (CDCl3 300MHz) | |
|---|---|
| 0,55 (s) | $CH_3$ en 18 |
| ~1,33 | OH |
| ~3,66 (m) | C<u>H</u>$_2$-OH |
| 4,41 (d) | H11 |
| 5,80 (s) | H4 |

<u>EXEMPLE 1</u> **: 3-hydroxy-11β-[4-[3-(1-pipéridinyl)propyl] phényl]-estra-1,3,5(10)-trièn-17-one**

<u>Stade A</u> : formation du mésylate

11β-[4-[3-[(méthylsulfonyl)oxy]propyl]phényl]-estra-4,9-diène-3,17-dione

A une solution de 3,41 g de 11β-[4-(3-hydroxypropyl)phényl]-estra-4,9-diène-3,17-dione préparé à la préparation 1 dans 30 ml de $CH_2Cl_2$, sous atmosphère inerte et à température ambiante, on ajoute 1,53 ml de TEA et 0,72 ml de chlorure de méthane sulfonyle en solution dans 2 ml de $CH_2Cl_2$ en maintenant la température à 0-5°C et agite 40 mn à cette température. Après lavage, séchage on évapore sous pression réduite jusqu'à obtention de 4,15 g de produit brut attendu.
F = 196°C
Rf ($CH_2Cl_2$/Acétone 8/2) : 0,51

| RMN (CDCl3) 300MHz | |
|---|---|
| 0,54 (s) | $CH_3$ en 18 |
| 3,00 (s) | $OSO_2C$<u>H</u>$_3$ |

(suite)

| RMN (CDCl3) 300MHz | |
|---|---|
| 4,21 (t) J=5,5 | $C\underline{H}_2$-$OSO_2CH_3$ |
| 4,41 (dl) J=7 | H11 |
| 5,80 (s) | H4 |
| 7,11 | H aromatiques |

Stade B : Aromatisation du cycle A

3-hydroxy-11β-[4-[3-[(méthylsulfonyl)oxy]propyl]phényl]-estra-1,3,5(10)-trièn-17-one

**a) Aromatisation**

A une solution de 4,15 g de la diénone préparée au stade précédent dans 40 ml de $CH_2Cl_2$, sous atmosphère inerte et à température ambiante, on ajoute, en refroidissant au bain de glace, 4 ml d'anhydride acétique et 2 ml de bromure d'acétyle et agite 1 heure.

**b) Saponification**

On évapore sous pression réduite, ajoute sous atmosphère inerte 20 ml de THF et ajoute en refroidissant au bain de glace, 20 ml de méthanol puis 28 ml de soude 2N. On agite pendant 40 mn, acidifie avec de l'acide chlorhydrique 2N, extrait à l'acétate d'éthyle, lave à l'eau salée puis évapore sous pression réduite jusqu'à obtention de 4,54 g de produit brut que l'on purifie par chromatographie en éluant avec le mélange $CH_2Cl_2$/AcOEt 9/1. On obtient 3,18 g de produit attendu et 590 mg d'un produit secondaire (3-céto-5(10), 9(11)-diène).
Rf ($CH_2Cl_2$/AcOEt 9/1) : 0,23

| RMN ($CDCl_3$) 300MHz | |
|---|---|
| 0,43 (s) | $CH_3$ en 18 |
| 2,92 (s) | $OSO_2C\underline{H}_3$ |
| 4,02 (m) | H11 |
| 4,12 (m) | $C\underline{H}_2$-$OSO_2$- |
| 4,61 (s) | OH en 3 |
| 6,41 (dd) | H2 |
| 6,61 | H4 |
| 6,81 (d) | H1 |
| 6,89 et 7,01 | H aromatiques |

Stade C : Introduction de l'amine

3-hydroxy-11β-[4-[3-(1-pipéridinyl)propyl]phényl]-estra-1,3,5(10)-trièn-17-one

A une solution de 1,45 g du mésylate préparé au stade B dans 15 ml de THF, sous atmosphère inerte et à température ambiante, on ajoute, 2,96 ml de pipéridine, porte au reflux pendant 3 h revient à la température ambiante, ajoute de l'acétate d'éthyle, lave au bicarbonate de sodium, à l'eau salée puis évapore sous pression réduite jusqu'à obtention de 1,48 g de produit brut que l'on purifie par chromatographie en éluant avec le mélange $CH_2Cl_2$/AcOEt/$NH_4OH$ 90/10/0,5. On obtient 1,2 g de produit attendu .
Rf ($CH_2Cl_2$/AcOEt/$NH_4OH$ 90/10/0,5) : 0,35

| RMN ($CDCl_3$) 300MHz | |
|---|---|
| 0,44 (s) | $CH_3$ en 18 |
| 1,59 | N-$CH_2$-$C\underline{H}_2$ du cycle |
| 2,41 | N-$C\underline{H}_2$-$CH_2$ du cycle |
| 4,02 (tl) | H11 |

(suite)

| RMN ($CDCl_3$) 300MHz | |
|---|---|
| 6,31 (dd) | H2 |
| 6,52 (d) | H4 |
| 6,77 (d) | H1 |
| ~6,81 et ~6,98 | H aromatiques |

**EXEMPLE 2 : 3-hydroxy-11β-[4-[3-(1-pyrrolidinyl)propyl] phényl]-estra-1,3,5(10)-trièn-17-one**

On opère comme à l'exemple 1 stade C à partir de 456 mg du mésylate préparé au stade B de l'exemple 1 et 0,79 ml de pyrrolidine. On obtient 441 mg de produit brut que l'on purifie par chromatographie en éluant avec le mélange AcOEt/TEA 7/3 puis par cristallisation dans de l'éther isopropylique. On obtient 347 mg de produit attendu . F = 180°C Rf ($CH_2Cl_2$/AcOEt/$NH_4OH$ 9/1/0,5) : 0,33

| RMN ($CDCl_3$) 300MHz | |
|---|---|
| 0,44 (s) | $CH_3$ en 18 |
| 1,75 | N-$CH_2$-$C\underline{H_2}$ du cycle |
| 2,50 | N-$C\underline{H_2}$-$CH_2$ du cycle |
| 4,03 (tl) | H11 |
| 6,25 (dd) | H2 |
| 6,51 (d) | H4 |
| 6,75 (d) | H1 |
| ~6,84 et ~6,99 | H aromatiques |

**EXEMPLE 3 : 3-hydroxy-11β-[4- [3-diméthylamino) propyl] phényl]-estra-1,3,5(10)-trièn-17-one**

On opère comme à l'exemple 1 mais en utilisant comme amine la diméthylamine.

**EXEMPLE 4 : 4-chloro-3-hydroxy-11β-[4-[3-(1-pipéridinyl) propyl]phényl]-estra-1,3,5(10)-trièn-17-one**

Stade A : chloration

4-chloro-11β-[4-(3-hydroxypropyl)phényl]-estra-4,9-dièn-3-one.

A une solution de 11,9 g de 11β-[4-(3-hydroxypropyl)phényl]-estra-4,9-diène-3,17-dione préparé à la préparation 1 dans 100 ml de DMF, sous atmosphère inerte et à 60°C, on ajoute 4,93 g de N-chloro succinimide et agite 10 mn à cette température. On verse dans l'eau, extrait, lave, sèche, on évapore sous pression réduite jusqu'à obtention de 16,2 g de produit brut attendu que l'on purifie par chromatographie en éluant avec le mélange $CH_2Cl_2$/Acétone 85/15. On obtient 9,34 g de produit pur attendu.
Rf ($CH_2Cl_2$/Acétone 85/15) : 0,3

| RMN ($CDCl_3$) 300MHz | |
|---|---|
| 0,56 (s) | $CH_3$ en 18 |
| 3,24 (dt) | H équatorial |
| 3,65 (t) | $C\underline{H_2}$-OH |
| 4,42 (d) | H11 |
| ~7,09 | H aromatiques |

Stade B : formation du mésylate

4-chloro-11β-[4-[3-[(méthylsulfonyl)oxy]propyl]phényl]-estra-4,9-dièn-3-one.

A une solution de 9,34 g de l'alcool préparé au stade A dans 90 ml de $CH_2Cl_2$, sous atmosphère inerte et à température ambiante, on ajoute 3,86 ml de TEA et 1,82 ml de chlorure de méthane sulfonyle en solution dans 5 ml de $CH_2Cl_2$ en maintenant la température à 0-5°C et agite 30 mn à cette température. Après lavage, séchage on évapore sous pression réduite jusqu'à obtention de 11 g de produit brut attendu.
Rf ($CH_2Cl_2$/Acétone 85/15) : 0,3

| RMN (CDCl3) 250MHz | |
|---|---|
| 0,56 (s) | $CH_3$ en 18 |
| 3,00 (s) | $OSO_2C\underline{H}_3$ |
| 3,26 (dt) | H équatorial |
| 4,22 (t) | $C\underline{H}_2$-$OSO_2CH_3$ |
| 4,42 (dl) | H11 |
| ~7,10 | H aromatiques |

Stade C : Aromatisation du cycle A

4-chloro-3-hydroxy-11β-[4-[3-[(méthanesulfonyl)oxy]propyl]phényl]-estra-1,3,5(10)-trièn-17-one

**a) Aromatisation**

A une solution de 10,37 g de la diènone préparée au stade précédent dans 100 ml de $CH_2Cl_2$, sous atmosphère inerte et à température ambiante, on ajoute, en refroidissant au bain de glace, 10 ml d'anhydride acétique et 2 ml de bromure d'acétyle et agite 6 heures à température ambiante.

**b) Saponification**

On évapore sous pression réduite, ajoute 50 ml de THF et ajoute, en refroidissant au bain de glace, 50 ml de méthanol puis 70 ml de soude 2N. On agite pendant 45 mn, acidifie avec 70 ml d'acide chlorhydrique 2N, extrait à l'acétate d'éthyle, lave à l'eau salée puis évapore sous pression réduite jusqu'à obtention de 11,5 g de produit brut que l'on purifie par chromatographie en éluant avec le mélange cyclohexane/AcOEt 9/1. On obtient 6 g de produit attendu (Rf=0,27) et 768 mg d'un produit secondaire (dérivé 4-chloro-1,3,5(10),9(11) tétraènone ne comportant pas d'hydroxy en 3 (Rf=0,39)).
Rf ($CH_2Cl_2$/AcOEt 9/1) : 0,27

| RMN ($CDCl_3$) 250MHz | |
|---|---|
| 0,43 (s) | $CH_3$ en 18 |
| 2,93 (s) | $OSO_2C\underline{H}_3$ |
| 4,02 (m) | H11 |
| 4,14 | $C\underline{H}_2$-$OSO_2$- |
| 5,46 (s) | OH en 3 |
| 6,64 | H2 |
| 6,81 | H1 |
| 6,92 | H aromatiques |

Stade D : Introduction de l'amine

4-chloro-3-hydroxy-11β-[4-[3-(1-pipéridinyl)propyl]phényl]-estra-1,3,5(10)-trièn-17-one

A une solution de 1,5 g du mésylate préparé au stade précédent dans 15 ml de THF, sous atmosphère inerte et à température ambiante, on ajoute, 2,96 ml de pipéridine, porte au reflux pendant 3 h revient à la température ambiante, ajoute de l'acétate d'éthyle, lave au bicarbonate de sodium, à l'eau salée puis évapore sous pression réduite

jusqu'à obtention de 1,66 g de produit brut que l'on purifie par chromatographie en éluant avec le mélange $CH_2Cl_2$/MeOH/$NH_4OH$ 90/10/0,5. On obtient 1,2 g de produit attendu .
Rf ($CH_2Cl_2$/AcOEt/$NH_4OH$ 90/10/0,5) : 0,27

| RMN ($CDCl_3$) 300MHz | |
|---|---|
| 0,43 (s) | $CH_3$ en 18 |
| 1,62 | N-$CH_2$-C$\underline{H_2}$ du cycle |
| 2,46 | N-C$\underline{H_2}$-$CH_2$ du cycle |
| 4,01 (tl) | H11 |
| 6,61 (d) | H2 |
| 6,79 (d) | H1 |
| ~6,89 | H aromatiques |

**<u>EXEMPLE 5</u> : 4-chloro-3-hydroxy-11β-[4-[3-(1-pyrrolidinyl) propyl]phényl]-estra-1,3,5(10)-trièn-17-one**

On opère comme à l'exemple 4 stade D mais à partir de 517 mg du mésylate (exemple 4 stade C) et 0,84 ml de pyrrolidine. On obtient 510 mg de produit brut que l'on purifie par chromatographie en éluant avec le mélange $CH_2Cl_2$/MeOH/$NH_4OH$ 90/10/1. On obtient 380 mg de produit attendu .
Rf ($CH_2Cl_2$/AcOEt/$NH_4OH$ 90/10/1) : 0,3

| RMN ($CDCl_3$) 300MHz | |
|---|---|
| 0,42 (s) | $CH_3$ en 18 |
| 1,75 | N-$CH_2$-C$\underline{H_2}$ du cycle |
| 2,46 | N-C$\underline{H_2}$-$CH_2$ du cycle du cycle |
| 4,02 (sl) | H11 |
| 6,56 (d) | H2 |
| 6,79 (d) | H1 |
| 6,89 (AA'BB') | H aromatique |

**<u>EXEMPLE 6</u> : 4-chloro-3-hydroxy-11β-[4-[3-(diéthylamino) propyl]phényl]-estra-1,3,5(10)-trièn-17-one**

On opère comme à l'exemple 4 stade D mais à partir de 517 mg du mésylate (exemple 4 stade c) et 1 ml de diéthylamine. On obtient 500 mg de produit brut que l'on purifie par chromatographie en éluant avec le mélange $CH_2Cl_2$/MeOH/$NH_4OH$ 90/10/1. On obtient 385 mg de produit attendu.
Rf ($CH_2Cl_2$/AcOEt/$NH_4OH$ 90/10/1) : 0,31

| RMN ($CDCl_3$) 300MHz | |
|---|---|
| 0,42 (s) | $CH_3$ en 18 |
| 0,95 | N-$CH_2$-C$\underline{H_3}$ |
| 2,49 | N-C$\underline{H_2}$-$CH_3$ |
| 4,02 (t) | H11 |
| 6,59 (d) | H2 |
| 6,80 (d) | H1 |
| 6,90 (AA'BB') | H aromatiques |

**<u>EXEMPLE 7</u> : 11β-[4-[3-(1-pyrrolidinyl)propyl]phényl]-estra-1,3,5(10)-triène-3,17β-diol**

A une solution de 310 mg du produit obtenu à l'exemple 2 dans 3 ml de méthanol, on ajoute à 0-5°C, 54 mg de borohydrure de sodium à 97 %, agite 1 heure à cette température, ajoute de l'eau salée, extrait, lave, sèche et évapore sous pression réduite jusqu'à obtention de 320 mg de produit brut que l'on purifie par chromatographie en éluant avec le mélange $CH_2Cl_2$/MeOH/$NH_4OH$ 90/10/1. On obtient 217 mg de produit pur attendu.
Rf ($CH_2Cl_2$/MeOH/$NH_4OH$) : 0,23

| RMN ($CDCl_3$) 300MHz | |
|---|---|
| 0,33 (s) | $CH_3$ en 18 |
| 1,76 | N-$CH_2$-$\underline{CH_2}$ du cycle |
| 2,52 | N-$\underline{CH_2}$-$CH_2$ du cycle |
| 3,69 (dd) | H17 |
| 3,96 (tl) | H11 |
| 6,27 (dd) | H2 |
| 6,47 (d) | H4 |
| 6,76 (d) | H1 |
| ~6,83 et ~6,98 | H aromatiques |

**<u>EXEMPLE 8</u> : 11β-[4-(3-diméthylamino)propyl)phényl]-estra-1,3,5(10)-triène-3,17β-diol**

On opère comme à l'exemple 7 (réduction avec le borohydrure de sodium) mais à partir du produit obtenu à l'exemple 3.
Rf AcOEt/iPrOH/$NH_4OH$ 70/30/1 : 0,17

| RMN ($CDCl_3$ + 1 goutte de $C_5D_5N$) 300MHz | |
|---|---|
| 0,32 (s) | $CH_3$ en 18 |
| 2,18 (s) | N-$\underline{CH_3}$ |
| 3,67 | H17 |
| 3,95 | H11 |
| 6,46 (dd) | H2 |
| 6,64 (d) | H4 |
| 6,81 (d) | H1 |
| ~6,88~7,00 | H aromatiques |

**<u>EXEMPLE 9</u> : 11β-[4-(1-pipéridinyl)propyl)phényl]-estra-1,3,5(10)-triène-3,17β-diol**

On opère comme à l'exemple 7 (réduction avec le borohydrure de sodium) mais à partir du produit obtenu à l'exemple 1. Rf acétate d'éthyle/TEA 90/10 : 0,30

| RMN ($CDCl_3$) 300MHz | |
|---|---|
| 0,33 (s) | $CH_3$ en 18 |
| 2,1 à 2,5 | N-$\underline{CH_2}$, Ph-$\underline{CH_2}$ |
| 3,70 (dd) | H17 |
| 3,96 (tl) | H11 |
| 6,32 (dd) | H2 |
| 6,47 (d) | H4 |
| 6,78 (d) | H1 |
| ~6,82~6,98 | H aromatiques |

**<u>EXEMPLE 10</u> : 4-chloro-11β-[4-[3-(1-pyrrolidinyl) propyl]phényl]-estra-1,3,5(10)-triène-3,17β-diol**

On opère comme à l'exemple 7 mais à partir de 257 mg de produit obtenu à l'exemple 5 et 42 mg de borohydrure de sodium à 97 %. On obtient 221 mg de produit brut que l'on purifie par cristallisation pour obtenir 154 mg de produit pur attendu.
Rf ($CH_2Cl_2$/MeOH/$NH_4OH$ 90/10/1) : 0,15

| RMN ($CDCl_3$) 300MHz | |
|---|---|
| 0,33 (s) | $CH_3$ en 18 |
| 1,73 (m) | N-$CH_2$-C$\underline{H}_2$ du cycle |
| ~2,37 ; ~2,50 | N-$CH_2$ et Ph-$CH_2$ de la chaîne |
| 2,43 (m) | N-C$\underline{H}_2$-$CH_2$ du cycle |
| 3,71 (dd) | H17 |
| 3,93 (tl) | H11 |
| 6,58 (d) | H2 |
| 6,76 (d) | H1 |
| 6,91 | H aromatiques |

**EXEMPLE 11 : 4-chloro-11β-[4-[3-(1-pipéridinyl) propyl]phényl]-estra-1,3,5(10)-triène-3,17β-diol**

On opère comme à l'exemple 7 mais à partir de 305 mg de produit obtenu à l'exemple 4 et 48 mg de borohydrure de sodium à 97 %. On obtient 275 mg de produit brut que l'on purifie par cristallisation dans l'acétone pour obtenir 170 mg de produit pur attendu.
F= 128°C
Rf ($CH_2Cl_2$/MeOH/$NH_4$OH 90/10/1) : 0,25

| RMN ($CDCl_3$) 300MHz | |
|---|---|
| 0,30 (s) | $CH_3$ en 18 |
| 1,55 (m) | N-$CH_2$-C$\underline{H}_2$ du cycle |
| 2,23 ; 2,41 | N-$CH_2$ et Ph-$CH_2$ de la chaîne |
| 2,36 (m) | N-C$\underline{H}_2$-$CH_2$ du cycle |
| 3,68 (dd) | H17 |
| 3,95 (t) | H11 |
| 6,58 (d) | H2 |
| 6,79 (d) | H1 |
| 6,88 | H aromatiques |

**EXEMPLE 12 : 4-chloro-11β-[4-[3-(diéthylamino) propyl]phényl]-estra-1,3,5(10)-triène-3,17β-diol**

On opère comme à l'exemple 7 mais à partir de 262 mg de produit obtenu à l'exemple 5 et 42 mg de borohydrure de sodium à 97 %. On obtient 231 mg de produit brut que l'on purifie par chromatographie pour obtenir 223 mg de produit pur attendu. Rf ($CH_2Cl_2$/MeOH/$NH_4$OH 90/10/1) : 0,18

| RMN ($CDCl_3$) 300MHz | |
|---|---|
| 0,30 (s) | $CH_3$ en 18 |
| 0,95 (t) | N-$CH_2$-C$\underline{H}_3$ |
| 2,49 (q) | N-C$\underline{H}_2$-$CH_3$ |
| 3,69 (t) | H17 |
| 3,95 (t) | H11 |
| 6,57 (d) | H2 |
| 6,78 (d) | H1 |
| 6,91 | H aromatiques |

**EXEMPLE 13 : 17α-méthyl-11β-[4-[3-(1-pipéridinyl)propyl]phényl]-estra-1,3,5(10)-triène-3,17β-diol**

On chauffe pendant 2 heures sous pression réduite 1,2 g de chlorure de cérium heptahydrate (CeCl3,7H2O) finement broyé, ramène à température ambiante sous gaz inerte, ajoute 12 ml de THF agite 2 heures à température ambiante puis ajoute à -68°C 1,9 ml d'une solution éthérée de méthyllithium. Après avoir agité pendant 30 mn à -72°C

on ajoute 300 mg du produit de l'exemple 1 en solution dans 3 ml de THF, laisse la température évoluer à température ambiante, filtre, lave, sèche et évapore sous pression réduite pour obtenir 325 mg de produit brut que l'on purifie par chromatographie en éluant avec le mélange $CH_2Cl_2$/MeOH/$NH_4OH$ 90/10/0,5.
Rf ($CH_2Cl_2$/MeOH/$NH_4OH$ 90/10/0,5) : 0,25

| RMN ($CDCl_3$) 300MHz | |
|---|---|
| 0,45(s) | $CH_3$ en 18 |
| 1,29 | $CH_3$ en 17 |
| 4,00 (tl) | H11 |
| 6,35 (dd) | H2 |
| 6,49 (d) | H4 |
| 6,79 (d) | H1 |
| ~6,81 et ~6,98 | H aromatiques |

**EXEMPLE 14 : 4-chloro-17α-méthyl-11β-[4-[3-(1-pipéridinyl)-propyl]phényl]-estra-1,3,5(10)-triène-3,17β-diol**

On opère comme à l'exemple 13 mais à partir de 300 mg du produit préparé à l'exemple 4 et 12 ml d'une solution éthérée de méthyllithium. On obtient 312 mg de produit brut que l'on purifie par chromatographie en éluant avec le mélange $CH_2Cl_2$/MeOH/$NH_4OH$ 90/10/1 pour obtenir 275 mg de produit pur attendu
Rf ($CH_2Cl_2$/MeOH/$NH_4OH$ 90/10/1) : 0,25

| RMN ($CDCl_3$) 300MHz | |
|---|---|
| 0,45 (s) | $CH_3$ en 18 |
| 1,29 (s) | $CH_3$ en 17 |
| 1,56 (m) | N-$CH_2$-C<u>H</u>$_2$- (cycle) |
| 2,34 (m) | N-C<u>H</u>$_2$-$CH_2$- (cycle) |
| 2,26 (m) et 2,46 (t) | $CH_2$-N et $CH_2$-Ph (chaîne) |
| 3,99 (tl) | H11 |
| 6,59 (d, J=8,5Hz) | H2 |
| 6,8 (d) | H1 |
| ~6,87 | H aromatiques |

**EXEMPLE 15 : 11β-[4-[3-(1-pipéridinyl)propyl]phényl]-17α-(trifluorométhyl)-estra-1,3,5(10)-triène-3,17β-diol**

**1) Préparation du dérivé 17β-triméthylsilyloxy, 17α-trifluorométhyle.**

On sèche pendant 2 heures à 120°C, 83 mg de fluorure de tétraméthylammonium tétrahydraté, ramène à température ambiante sous gaz inerte et ajoute 236 mg du produit de l'exemple 1, 3 ml de THF, et 0,3 ml de trimethyl (trifluoromethyl) silane ($CF_3SiMe_3$) en maintenant la température à 0-5°C. On laisse la température évoluer à 10°C et agite 2 heures 30 à 0-5°C, puis on verse dans l'eau salée, extrait, lave, sèche et évapore sous pression réduite pour obtenir 340 mg de produit brut.

**2) Déprotection de l'alcool.**

On ajoute à une solution de 340 mg du produit brut obtenu précédemment dans 4 ml de THF, sous atmosphère inerte et à température ambiante, 2 ml de fluorure de tétrabutylammonium en solution 1M dans le THF, agite 2 heures à température ambiante, verse dans de l'eau, extrait, lave, sèche et évapore sous pression réduite jusqu'à obtention de 530 mg de produit brut que l'on purifie par chromatographie en éluant avec le mélange $CH_2Cl_2$/MeOH/$NH_4OH$ 90/10/1 puis avec le mélange AcOEt/TEA 95/5. On obtient 96 mg de produit pur attendu.
Rf (AcOEt/TEA 95/5 ) : 0,25

| RMN ($CDCl_3$) 300MHz | |
|---|---|
| 0,50 (s) | $CH_3$ en 18 |

(suite)

| RMN ($CDCl_3$) 300MHz | |
|---|---|
| 4,03 (tl) | H11 |
| 6,36 (dd) | H2 |
| 6,52 (dd) | H4 |
| 6,78 (d) | H1 |
| ~6,85 et ~6,96 | H aromatiques |

**EXEMPLE 16 : (17R) 11β-[4-[3-(diméthylamino)propyl)phényl]-spiro-(estra-1,3,5(10)-triène-17,2'(5'H)-furan)-3-ol**

On opère comme à l'exemple 1 mais à partir de diméthylamine et de 11β-[4-(3-hydroxypropyl)phényl]-spiro-(estra-4,9-diène--17,2'(5'H)-furan)-3-one.

**EXEMPLE 17 : (17R) 4',5'-dihydro-11β-[4-[3-(diméthylamino) propyl]phényl]-spiro-(estra-1,3,5(10)-triène-17,2'(3'H)-furan)-3-ol**

La réduction s'effectue par hydrogénation avec du palladium à 10 % sur charbon du produit de l'exemple 16. Rf (AcOEt/TEA 8/2) : 0,25

| RMN ($CDCl_3$) 300MHz | |
|---|---|
| 0,46 (s) | $CH_3$ en 18 |
| 2,20 (s) | N-$C\underline{H}_3$ |
| 3,76 (m) | H'3 |
| 3,98 (m) | H11 |
| 6,32(dd) | H2 |
| 6,47 (d) | H4 |
| 6,78 (d) | H1 |
| ~6,83~6,97 | H aromatiques |

**Tests pharmacologiques**

**1 - Effet sur la prolifération de cellules mammaires**

L'activité proliférative des molécules est étudiée comparativement à celle de l'oestradiol sur les cellules mammaires humaines MCF-7 en culture.

Pour mettre en évidence un effet agoniste de l'oestradiol et/ou des molécules testées, le milieu de culture d'entretien des cellules (riche en facteurs de croissance et en stéroïdes) est remplacé par un milieu appauvri, entre autres dépourvu de stéroïdes (DMEM supplémenté par 5 % de sérum déstéroïdé et sans rouge de phénol) . Les cellules subissent ce sevrage deux jours avant le début de l'essai.

Après 7 jours de culture en présence des produits à étudier, la prolifération cellulaire est évaluée par dosage du DNA. Dans chaque essai, l'effet de l'oestradiol à $10^{-10}$M (croissance cellulaire en présence d'oestradiol moins croissance cellulaire en présence du solvant) détermine le 100 % de l'activité agoniste. L'activité des molécules est évaluée en comparaison à ce témoin interne. Les molécules induisant une croissance cellulaire identique à celle observée avec le solvant seul sont classées "inactives", celles induisant une croissance cellulaire inférieure à celle observée avec le solvant sont classées "inhibiteur".

| | ACTIVITE |
|---|---|
| Estradiol | Agoniste |
| Exemple 12 | Inhibiteur |
| Exemple 7 | Inhibiteur |
| Exemple 9 | Inhibiteur |

(suite)

| | ACTIVITE |
|---|---|
| Exemple 13 | Inhibiteur |
| Exemple 11 | Mixte |
| * Mixte : légère activité agoniste aux très faibles concentrations et activité inhibitrice aux concentrations plus fortes. | |

Conclusion :

Les produits testés ne sont pas agonistes de la croissance des cellules MCF-7, certains sont même inhibiteurs de celle-ci.

**2 - Affinité au récepteur estrogène humain (REH)**

Un extrait cytosolique de cellules SF9 contenant le récepteur oestrogène humain recombinant est obtenu par surexpression dans un système cellules d'insectes-Baculovirus, selon la méthodologie générale décrite par N.R. WEBB et al. (Journal of Methods in Cell and Molecular Biology, (1990) vol 2 n° 4, 173-188) et dont l'application est décrite pour l'expression des récepteurs hormonaux humains, par exemple le récepteur glucocorticoïde humain (G. SRINIVASAN et al. Molecular Endocrinology (1990) vol 4 n° 2 209-216).

On utilise le kit BaculoGold Transfection Kit (PharMingen, référence 21000K) pour générer le baculovirus recombinant contenant le fragment d'ADNc décrit dans le vecteur d'expression HEGO par L. TORA et al. (The EMBO Journal (1989) vol 8 n° 7 1981-1986), comprenant la région codante pour le récepteur estrogène humain de type sauvage avec une glycine en position 400.

Le virus recombinant ainsi obtenu est utilisé pour exprimer le récepteur progestogène dans les cellules d'insectes SF9 (ATCC CRL1711), selon la méthodologie connue citée précédemment.

$2 \times 10^7$ cellules SF9 sont cultivées dans un flacon "Falcon" de 175 $cm^2$ dans le milieu TNM-FH "SIGMA" supplémenté avec 10 % de sérum de veau foetal (SVF) et avec 50 microgrammes/ml de gentamycine. Après infection puis incubation à 27°C pendant 40 à 42 heures, les cellules sont lysées dans 1 ml de tampon de lyse (Tris 20 mM-HCl pH8, EDTA 0,5 mM, DTT 2 mM, Glycérol 20 %, KCl 400 mM) par un cycle de congélation-décongélation que l'on répète encore deux fois. Le surnageant, contenant le récepteur estrogène humain recombinant est conservé dans l'azote liquide par dose de 0,5 ml.

Le surnageant est incubé à 0°C pendant 24 heures avec une concentration constante (T) d'oestradiol tritié en présence de concentrations croissantes soit d'estradiol froid ($0\text{-}1000 \times 10^{-9}$M), soit du produit froid à tester ($0 - 25000 \times 10^{-9}$M). La concentration d'estradiol tritié liée (B) est ensuite mesurée dans chaque incubat par la technique d'adsorption au charbon dextran.

**3 - Calcul de l'affinité relative de liaison (ARL)**

On trace les 2 courbes suivantes : le pourcentage de l'hormone tritiée liée 100xB/BO en fonction du logarithme de la concentration de l'hormone de référence froide ou en fonction du logarithme de la concentration du produit froid testé.

On détermine la droite d'équation suivante : $I_{50} = 100 (B_0/B_0+Bmin/B_0)/2 = 100 (1+Bmin/B_0)=50(1+Bmin/B_0)$

$B_0$ = Concentration de l'hormone tritiée liée en l'absence de tout produit froid,

B = Concentration de l'hormone tritiée liée en présence d'une concentration X de produit froid,

Bmin = Concentration de l'hormone tritiée liée pour une incubation de cette hormone tritiée à la concentration (T) en présence d'un grand excès d'hormone froide de référence ($1000 \times 10^{-9}$M) pour récepteur humain.

Les intersections de la droite $I_{50}$ et des courbes, permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminé par l'équation :

$$ARL = 100 (CH) / (CX)$$

Les résultats obtenus sont les suivants :

| EXEMPLES | EH estradiol = 100 24 H |
|---|---|
| 12 | 28 |
| 7 | 59 |
| 9 | 28 |
| 13 | 42 |
| 11 | 14 |

Conclusion :

Les produits testés présentent de bonnes affinités pour le récepteur estrogène humain.

## Revendications

**1.** Composés de formule générale (I) :

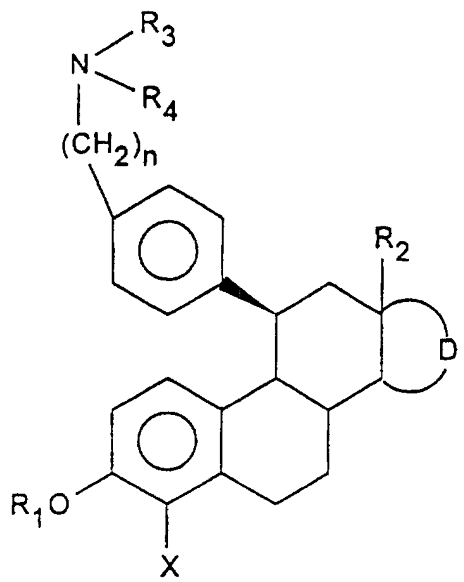

(I)

dans laquelle :

$R_1$ représente un atome d'hydrogène, un radical $(CH_2)_m$-Ar, (CO)-Ar, $(CH_2)_m$-Alk ou (CO)-Alk,
$R_2$ représente un radical dérivé d'un hydrocarbure, linéaire ou ramifié, saturé ou insaturé renfermant de 1 à 6 atomes de carbone
D représente le reste d'un cycle pentagonal ou hexagonal éventuellement substitué et éventuellement porteur d'insaturation,
X représente un atome d'halogène ou un atome d'hydrogène,
n est égal à 3, 4 ou 5,
soit $R_3$ et $R_4$ identiques ou différents représentent un atome d'hydrogène, un groupement $(CH_2)_m$-Ar, $(CH_2)_m$-Het ou $(CH_2)_m$-Alk,
soit $R_3$ et $R_4$ forment ensemble avec l'atome d'azote auquel ils sont liés un hétérocycle mono ou polycyclique, saturé ou insaturé, aromatique ou non aromatique, de 3 à 15 chaînons renfermant éventuellement de 1 à 3 hétéroatomes additionnels choisis parmi l'oxygène, le soufre et l'azote, non substitué ou substitué,
Ar représentant un groupement aryle carbocyclique renfermant de 6 à 18 atomes de carbone, Het représentant un hétérocycle aromatique ou non aromatique, saturé ou non saturé, comportant de 1 à 9 atomes de carbone

et de 1 à 5 hétéroatomes choisis parmi les atomes d'oxygène, d'azote ou de soufre, Alk représentant un radical dérivé d'un hydrocarbure, non aromatique, linéaire, ramifié ou cyclique, saturé ou insaturé et comportant de 1 à 12 atomes de carbone, les radicaux Ar, Het ou Alk pouvant être substitués ou non substitués, m représente 0, 1, 2 ou 3, ainsi que leurs sels d'addition avec les bases ou les acides.

**2.** Composés de formule générale (I) telle que définie à la revendication 1, dans laquelle D représente le reste d'un cycle pentagonal de formule :

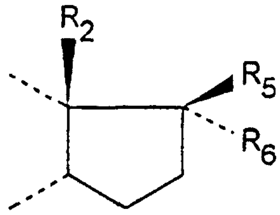

dans laquelle $R_2$ conserve la même signification qu'à la revendication 1,
soit $R_5$ représente un radical OH, O-$(CH_2)_m$-Alk, O-(CO)-Alk, O-$(CH_2)_m$-Ar, O-(CO)-Ar, O-$(CH_2)_m$-Het, O-(CO)-Het et $R_6$ représente un atome d'hydrogène, un radical alkyle, alkényle ou alkynyle renfermant de 1 à 6 atomes de carbone substitué ou non substitué, m, Alk, Ar et Het étant tels que définis à la revendication 1,
soit $R_5$ et $R_6$ forment ensemble avec l'atome de carbone qui les porte l'un des cycles suivants :

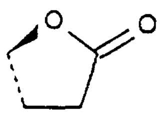

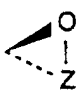

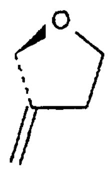

dans lequel Z représente un groupement -$(CH_2)_l$- ou -CH=CH-$(CH_2)_{l'}$, l étant un entier compris entre 1 et 4 et l' étant un entier égal à 1 ou 2,
soit $R_5$ et $R_6$ forment ensemble un groupement oxo,
ainsi que leurs sels d'addition avec les acides ou les bases.

**3.** Composés de formule générale (I) telle que définie à la revendication 1 ou 2, répondant à la formule générale (I') :

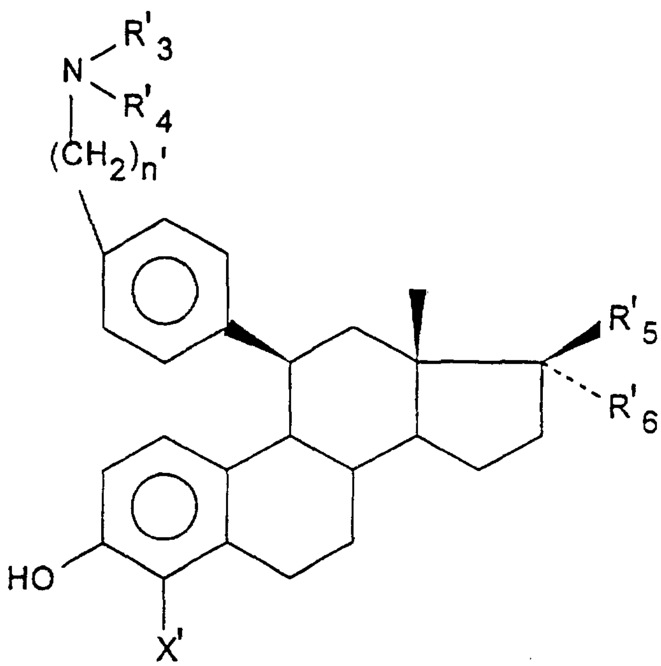

(I')

dans laquelle :

X' représente un atome de chlore, de brome ou d'hydrogène, n' est égal à 3,
soit $R'_3$ et $R'_4$ identiques ou différents représentent un radical alkyle renfermant de 1 à 6 atomes de carbone
soit $R'_3$ et $R'_4$ forment ensemble, avec l'atome d'azote auquel ils sont liés, un reste mono ou polyclique saturé de 3 à 15 chaînons renfermant éventuellement un hétéroatome additionnel choisi parmi l'oxygène, le soufre et l'azote,
$R'_5$ et $R'_6$ ont la même signification que $R_5$ et $R_6$, selon la revendication 2 ainsi que leurs sels d'addition avec les acides et les bases.

**4.** Composés de formule générale (I') telle que définie à la revendication 3, dans laquelle :

soit $R'_5$ représente un radical OH et $R'_6$ représente un atome d'hydrogène, un radical alkyle, alkényle ou alkynyle renfermant de 1 à 6 atomes de carbone, substitué ou non substitué,
soit $R'_5$ et $R'_6$ forment ensemble avec l'atome de carbone qui les porte l'un des cycles suivants :

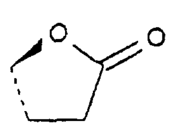 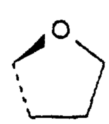 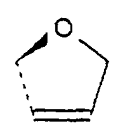 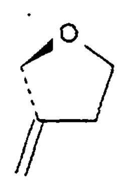

soit $R'_5$ et $R'_6$ forment ensemble un groupement oxo,

ainsi que leurs sels d'addition avec les acides ou les bases.

**5.** Composés de formule générale (I') telle que définie à la revendication 3 ou 4, dans laquelle :

X' représente un atome de chlore ou d'hydrogène,
soit $R'_3$ et $R'_4$ identiques ou différents représentent un radical alkyle renfermant de 1 à 6 atomes de carbone,
soit $R'_3$ et $R'_4$ forment ensemble avec l'atome d'azote les hétérocycles saturés suivants :

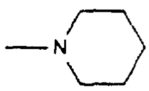 , 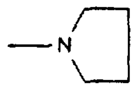 , 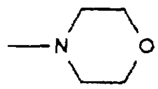 , 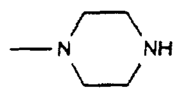 et 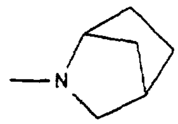

et soit $R'_5$ représente un radical OH et $R'_6$ représente un atome d'hydrogène, un radical alkyle, alkényle ou alkynyle renfermant de 1 à 6 atomes de carbone, substitué ou non substitué,
soit $R'_5$ et $R'_6$ forment ensemble avec l'atome de carbone qui les porte l'un des cycles suivants :

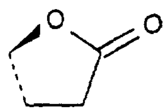 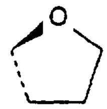 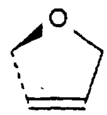 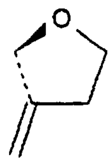

soit $R'_5$ et $R'_6$ forment ensemble un groupement oxo,

ainsi que leurs sels d'addition avec les acides ou les bases.

6. Composés de formule générale (I) telle que définie à l'une quelconque des revendications 1 à 5, dans laquelle X est un atome d'hydrogène.

7. Composés de formule générale (I) telle que définie à l'une quelconque des revendications 1 à 5, dans laquelle X est un atome de chlore.

8. Composés de formule (I) ou (I') telle que définie à l'une quelconque des revendications 1 à 7, dont les noms suivent :

3-hydroxy-11β-[4-[3-(1-pipéridinyl)propyl]phényl]-estra-1,3,5(10)-trièn-17-one,
3-hydroxy-11β-[4-[3-(1-pyrrolidinyl)propyl]phényl]-estra-1,3,5(10)-trièn-17-one,
3-hydroxy-11β-[4-[3-diméthylamino)propyl]phényl]-estra-1,3,5(10)-trièn-17-one,
4-chloro-3-hydroxy-11β-[4-[3-(1-pipéridinyl)propyl]phényl]-estra-1,3,5(10)-trièn-17-one
4-chloro-3-hydroxy-11β-[4-[3-(1-pyrrolidinyl)propyl]phényl]-estra-1,3,5(10)-trièn-17-one
4-chloro-3-hydroxy-11β-[4-[3-(diéthylamino)propyl]phényl]-estra-1,3,5(10)-trièn-17-one
11β-[4-[3-(1-pyrrolidinyl)propyl]phényl]-estra-1,3,5(10)-triène-3,17β-diol,
11β-[4-(3-diméthylamino)propyl)phényl]-estra-1,3,5(10)-triène-3,17β-diol
11β-[4-(1-pipéridinyl)propyl)phényl]-estra-1,3,5(10)-triène-3,17β-diol
4-chloro-11β-[4-[3-(1-pyrrolidinyl)propyl]phényl]-estra-1,3,5(10)-triène-3,17β-diol
4-chloro-11β-[4-[3-(1-pipéridinyl)propyl]phényl]-estra-1,3,5(10)-triène-3,17β-diol
4-chloro-11β-[4-[3-(diéthylamino)propyl]phényl]-estra-1,3,5(10)-triène-3,17β-diol
17α-méthyl-11β-[4-[3-(1-pipéridinyl)propyl]phényl]-estra-1,3,5(10)-triène-3,17β-diol,
4-chloro-17α-méthyl-11β-[4-[3-(1-pipéridinyl)propyl]phényl]-estra-1,3,5(10)-triène-3,17β-diol,
11β-[4-[3-(1-pipéridinyl)propyl]phényl]-17α-(trifluorométhyl)-estra-1,3,5(10)-triène-3,17β-diol
(17R) 11β-[4-[3-(diméthylamino)propyl]phényl]-spiro-(estra-1,3,5(10)-triène-17,2'(5'H)-furan)-3-ol
(17R) 4',5'-dihydro-11β-[4-[3-(diméthylamino)propyl]phényl]-spiro-(estra-1,3,5(10)-triène-17,2'(3'H)-furan)-3-ol.

9. Procédé de préparation des composés de formule (I) telle que définie à la revendication 1, dans lequel on soumet un composé de formule (II) :

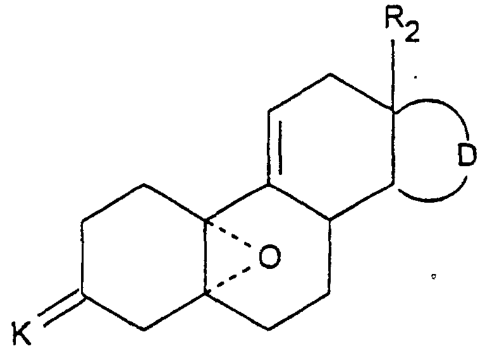

(II)

dans laquelle D et $R_2$ sont tels que définis à la revendication 1 et K représente un groupement protecteur de la fonction 3-céto, successivement aux réactions suivantes :

a) action d'un composé de formule (III) :

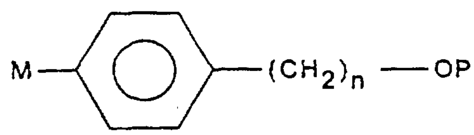

dans laquelle, M représente un dérivé métallique, P représente un groupement protecteur, de l'alcool et n est un entier égal à 3, 4 ou 5, puis le cas échéant déprotection d'une ou de plusieurs fonctions réactives protégées, afin d'obtenir un composé de formule ($III_a$) :

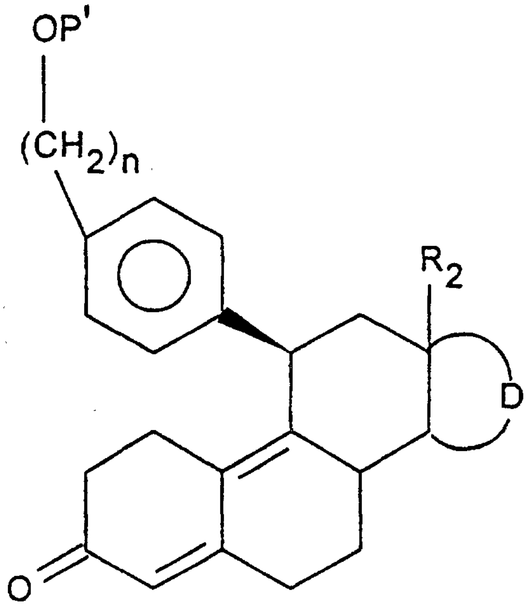

($III_a$)

P' ayant les mêmes valeurs que P ainsi que hydrogène,

b) action, le cas échéant, d'un réactif d'halogénation, afin d'obtenir un composé de formule ($III_b$) :

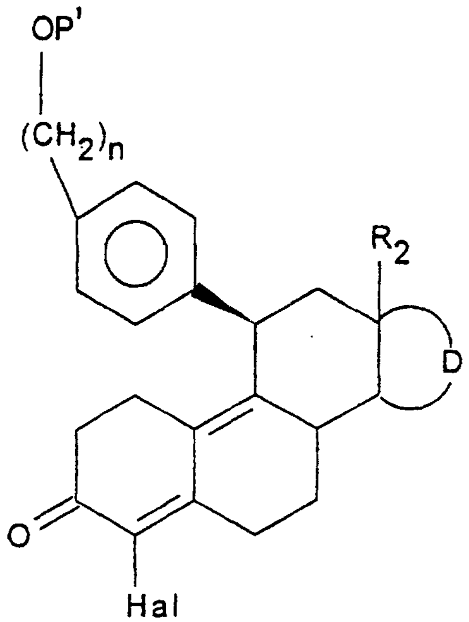

$(III_b)$

Hal représentant un atome d'halogène,

c) action d'un réactif d'aromatisation du cycle (A) sur les composés de formules $(III_a)$ et $(III_b)$, après avoir, le cas échéant, protégé et/ou activé la fonction OH, puis action d'une base pour obtenir le composé de formule (IV) :

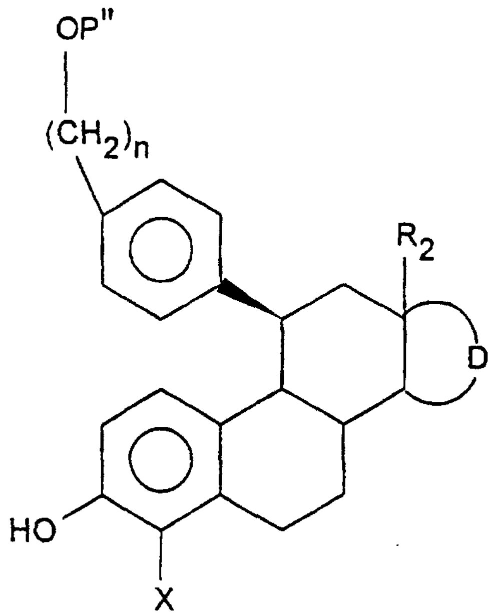

(IV)

P" ayant les mêmes valeurs que P' et pouvant représenter en outre un groupement activant,

X étant tel que défini à la revendication 1,

d) action d'une amine de formule (V) :

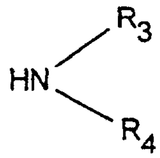

(V)

$R_3$ et $R_4$ étant tels que définis à la revendication 1, ce composé étant éventuellement sous forme de sel, afin d'obtenir certains des composés de formule (I), les composés de formules ($III_a$), ($III_b$), (IV) et (I) étant soumis si désiré ou si nécessaire, dans un ordre approprié, à l'une ou plusieurs des réactions suivantes :

- protection/déprotection du ou des groupements OH,
- alkylation/acylation du ou des groupements OH,
- action d'un agent de réduction lorsque D représente le reste d'un cycle pentagonal tel que défini à la revendication 2 et $R_5$ et $R_6$ forment ensemble un groupement oxo,
- action d'un organométallique ou de $CF_3SiMe_3$ sur les composés de formule (IV) ou (I) avec D représentant le reste d'un cycle pentagonal tel que défini à la revendication 2 et $R_5$ et $R_6$ forment ensemble un groupement oxo,
- action d'un agent de lactonisation sur les composés de formule (IV) ou (I) avec D représentant le reste d'un cycle pentagonal tel que défini à la revendication 2 et $R_5$ et $R_6$ formant ensemble un groupement oxo,
- action d'un agent de réduction de la double liaison, lorsque D représente le reste d'un cycle pentagonal tel que défini à la revendication 2 et $R_5$ et $R_6$ forment ensemble avec le carbone qui les porte, un groupement $O-(CH_2)_{l'}-CH=CH-$,
- action d'un agent de réduction de la double liaison, lorsque D représente le reste d'un cycle pentagonal tel que défini à la revendication 2, et $R_6$ est un radical alkényle ou alkynyle renfermant de 2 à 6 atomes de carbone,
- salification.

**10.** Procédé de préparation selon la revendication 9, des composés de formule générale (I') telle que définie à la revendication 3, dans lequel on soumet un composé de formule (II') :

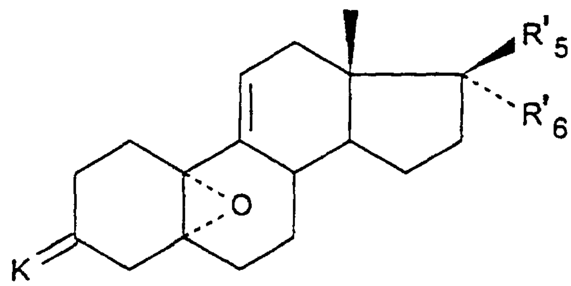

(II')

dans laquelle K, $R'_5$ et $R'_6$ sont tels que définis à la revendication 3, ou dans laquelle $R'_5$ est un radical CN et $R'_6$ est un hydroxyle protégé, successivement aux réactions suivantes :

a) action d'un composé de formule (III') :

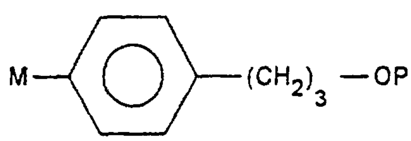

(III')

dans laquelle M et P sont tels que définis précédemment, puis déprotection d'une ou de plusieurs fonctions réactives protégées, afin d'obtenir un composé de formule ($III'_a$) :

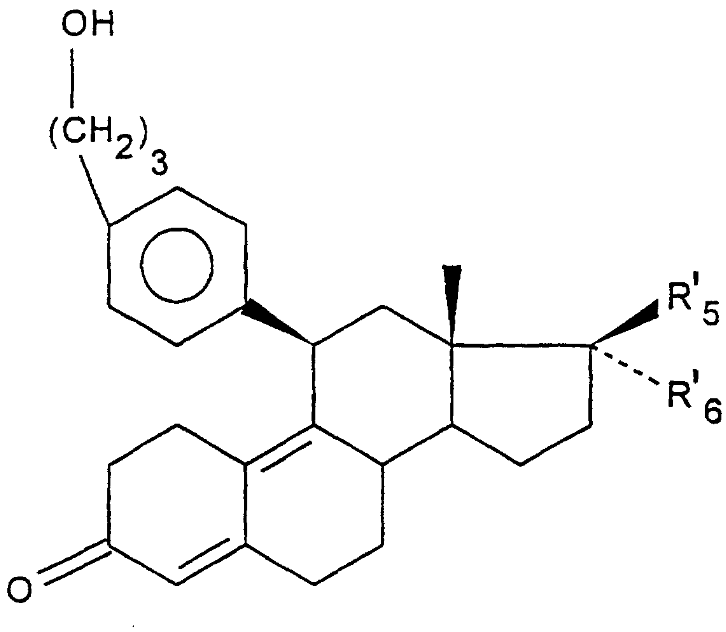

(III'a)

b) action, le cas échéant, d'un réactif d'halogénation afin d'obtenir un composé de formule ($III'_b$) :

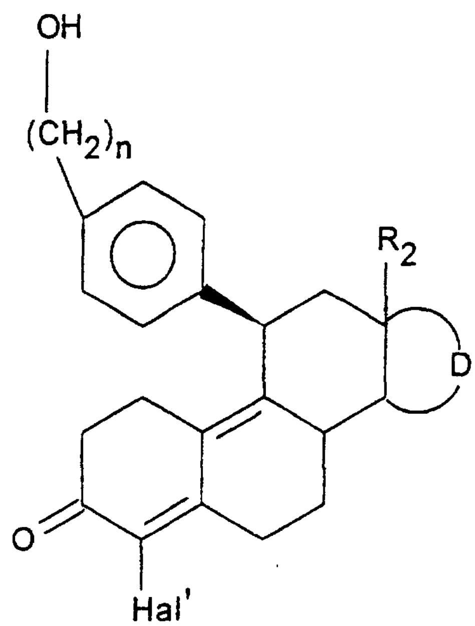

(III'b)

Hal' représentant un atome de chlore ou de brome,

c) activation de la fonction OH puis action d'un réactif d'aromatisation du cycle (A) sur les composés de formule ($III_a$) ou ($III_b$), puis action d'une base pour obtenir les composés de formule (IV') :

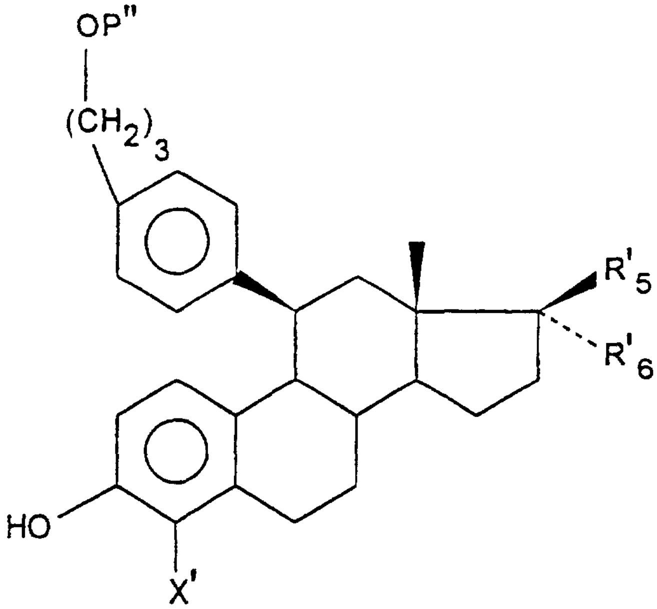

(IV')

X' et P" étant tels que définis à la revendication 9,
d) action d'une amine de formule (V') :

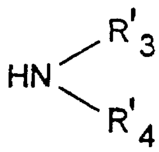

(V')

$R'_3$ et $R'_4$ étant tels que définis précédemment afin d'obtenir certains des composés de formule (I'), les composés de formules ($III'_a$), ($III'_b$), (IV') et (I') étant soumis, si désiré ou si nécessaire à l'une ou plusieurs des réactions suivantes :

- protection/déprotection du ou des groupements OH,
- alkylation/acylation du ou des groupements OH,
- action d'un agent de réduction lorsque $R'_5$ et $R'_6$ forment ensemble un groupement oxo,
- action d'un organométallique ou de $CF_3SiMe_3$ sur les composés de formule (IV') ou (I') avec $R'_5$ et $R'_6$ formant ensemble un groupement oxo,
- action d'un agent de lactonisation sur les composés de formule (IV') ou (I') avec $R'_5$ et $R'_6$ formant ensemble un groupement oxo,
- action d'un agent de réduction de la double liaison, lorsque $R'_5$ et $R'_6$ forment ensemble avec le carbone qui les porte, un groupement O-$(CH_2)_{l'}$ -CH=CH-,
- action d'un agent de réduction de la double liaison, lorsque $R'_6$ est un radical alkényle ou alkynyle renfermant de 2 à 6 atomes de carbone,
- salification.

**11.** A titre de médicaments les composés de formule (I) telle que définie à la revendication 1, ainsi que leurs sels d'addition avec les acides ou les bases pharmaceutiquement acceptables.

**12.** A titre de médicaments les composés de formule (I) ou (I') telle que définie à l'une quelconque des revendications 2 à 7, ainsi que leurs sels d'addition avec les acides ou les bases pharmaceutiquement acceptables.

**13.** A titre de médicaments les composés tels que définis à la revendication 8, ainsi que leurs sels d'addition avec les acides pharmaceutiquement acceptables.

**14.** Compositions pharmaceutiques renfermant un ou plusieurs des médicaments tels que définis à l'une quelconque des revendications 11, 12 ou 13.

**15.** A titre de produits intermédiaires nouveaux, les composés de formules générales ($III_a$), ($III_b$), ($III'_a$), ($III'_b$), (IV) ou (IV') telles que définies à la revendication 9 ou 10.

**Claims**

**1.** Compounds of general formula (I):

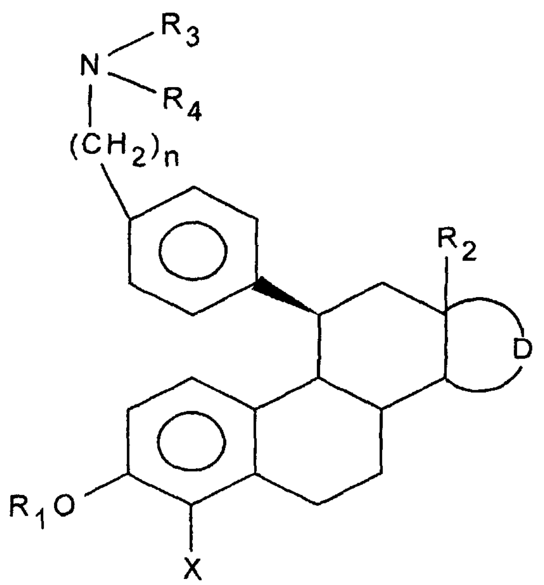

(I)

in which:

$R_1$ represents a hydrogen atom, a $(CH_2)_m$-Ar, (CO)-Ar, $(CH_2)_m$-Alk or (CO)-Alk radical,
$R_2$ represents a radical derived from a linear or branched, saturated or unsaturated hydrocarbon containing 1 to 6 carbon atoms
D represents the remainder of a pentagonal or hexagonal ring optionally substituted, and optionally an unsaturation carrier,
X represents a halogen atom or a hydrogen atom,
n is equal to 3, 4 or 5,
either $R_3$ and $R_4$, identical or different, represent a hydrogen atom, a $(CH_2)_m$-Ar, $(CH_2)_m$-Het or $(CH_2)_m$-Alk group,
or $R_3$ and $R_4$ form together with the nitrogen atom to which they are linked a mono- or polycyclic, saturated or unsaturated, aromatic or non aromatic heterocycle with 3 to 15 members optionally containing 1 to 3 additional heteroatoms chosen from oxygen, sulphur and nitrogen, non substituted or substituted,
Ar representing a carbocyclic aryl group containing 6 to 18 carbon atoms, Het representing an aromatic or non aromatic, saturated or unsaturated heterocycle containing 1 to 9 carbon atoms and 1 to 5 heteroatoms chosen from oxygen, nitrogen or sulphur atoms, Alk representing a non aromatic, linear, branched or cyclic, saturated or unsaturated radical derived from a hydrocarbon and comprising 1 to 12 carbon atoms, the Ar, Het or Alk radicals being able to be substituted or non substituted, m represents 0, 1, 2 or 3, as well as their addition salts with bases or acids.

**2.** Compounds of general formula (I) as defined in claim 1, in which D represents the remainder of a pentagonal ring of formula:

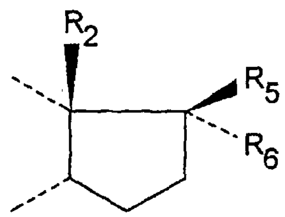

in which $R_2$ retains the same meaning as in claim 1,
either $R_5$ represents an OH, O-$(CH_2)_m$-Alk, O-(CO)-Alk, O-$(CH_2)_m$-Ar, O-(CO)-Ar, O-$(CH_2)_m$-Het, O-(CO)-Het radical and $R_6$ represents a hydrogen atom, an alkyl, alkenyl or alkynyl radical containing 1 to 6 carbon atoms, substituted or non substituted, m, Alk, Ar and Het being as defined in claim 1,
or $R_5$ and $R_6$ form together with the carbon atom which carries them one of the following rings:

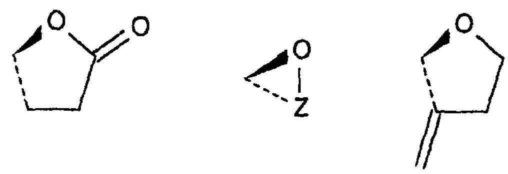

in which Z represents a $-(CH_2)_1-$ or $-CH=CH-(CH_2)_{1'}$ group, l being an integer comprised between 1 and 4 and 1' being an integer equal to 1 or 2,
or $R_5$ and $R_6$ form together an oxo group,
as well as their addition salts with acids or bases.

**3.** Compounds of general formula (I) as defined in claim 1 or 2, corresponding to general formula (I'):

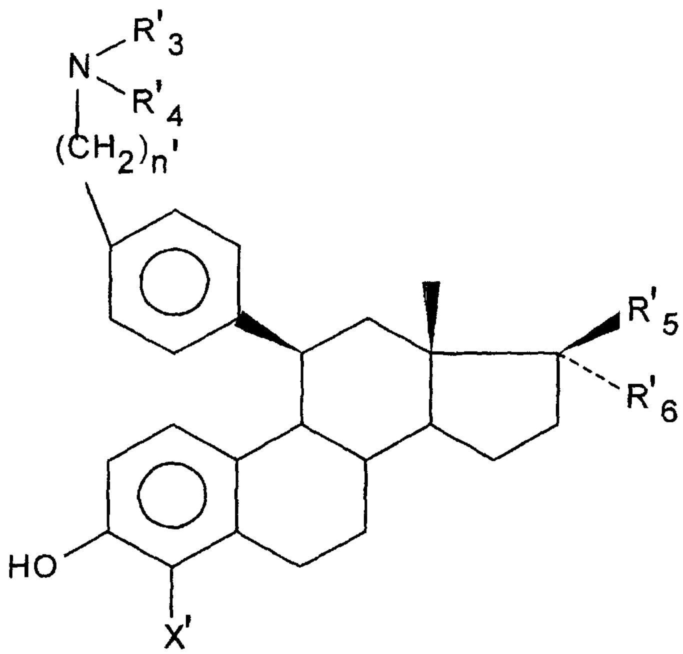

(I')

in which:

X' represents a chlorine, bromine or hydrogen atom,

n' is equal to 3,
either $R'_3$ and $R'_4$, identical or different, represent an alkyl radical containing 1 to 6 carbon atoms
or $R'_3$ and $R'_4$ form together with the nitrogen atom to which they are linked a saturated mono- or polycyclic remainder
with 3 to 15 members, optionally containing an additional heteroatom chosen from oxygen, sulphur and nitrogen,
$R'_5$ and $R'_6$ have the same meaning as $R_5$ and $R_6$ according to claim 2 as well as their addition salts with acids and bases.

**4.** Compounds of general formula (I') as defined in claim 3, in which:

either $R'_5$ represents an OH radical and $R'_6$ represents a hydrogen atom, an alkyl, alkenyl or alkynyl radical containing 1 to 6 carbon atoms, substituted or non substituted,
or $R'_5$ and $R'_6$ form together with the carbon atom which carries them one of the following rings:

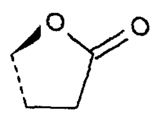 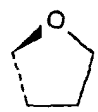 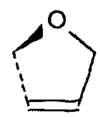 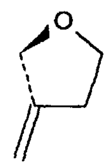

or $R'_5$ and $R'_6$ form together an oxo group,

as well as their addition salts with acids or bases.

**5.** Compounds of general formula (I') as defined in claim 3 or 4 in which:

X' represents a chlorine or hydrogen atom,
either $R'_3$ and $R'_4$ identical or different represent an alkyl radical containing 1 to 6 carbon atoms,
or $R'_3$ and $R'_4$ form together with the nitrogen atom the following saturated heterocycles:

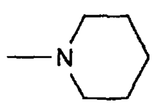 , 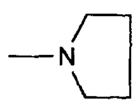 , 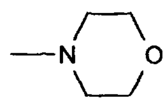 , 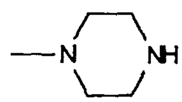 and 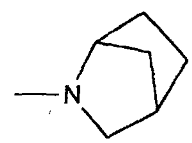

and either $R'_5$ represents an OH radical and $R'_6$ represents a hydrogen atom, an alkyl, alkenyl or alkynyl radical containing 1 to 6 carbon atoms, substituted or non substituted,
or $R'_5$ and $R'_6$ form together with the carbon atom which carries them one of the following rings:

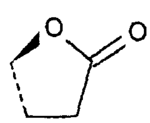 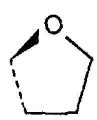 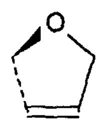 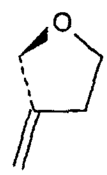

or $R'_5$ and $R'_6$ form together an oxo group,
as well as their addition salts with acids or bases.

**6.** Compounds of general formula (I) as defined in any one of claims 1 to 5 in which X is a hydrogen atom.

7. Compounds of general formula (I) as defined in any one of claims 1 to 5 in which X is a chlorine atom.

8. Compounds of formula (I) or (I') as defined in any one of claims 1 to 7 the names of which follow:

3-hydroxy-11β-[4-[3-(1-piperidinyl)propyl]phenyl]-estra-1,3,5(10)-trien-17-one,
3-hydroxy-11β-[4-[3-(1-pyrrolidinyl)propyl]phenyl]-estra-1,3,5(10)-trien-17-one,
3-hydroxy-11β-[4-[3-dimethylamino)propyl]phenyl]-estra-1,3,5(10)-trien-17-one,
4-chloro-3-hydroxy-11β-[4-[3-(1-piperidinyl)propyl]phenyl]-estra-1,3,5(10)-trien-17-one
4-chloro-3-hydroxy-11β-[4-[3-(1-pyrrolidinyl)propyl]phenyl]-estra-1,3,5(10)-trien-17-one
4-chloro-3-hydroxy-11β-[4-[3-(diethylamino)propyl]phenyl]-estra-1,3,5(10)-trien-17-one
11β-[4-[3-(1-pyrrolidinyl)propyl]phenyl]-estra-1,3,5(10)-triene-3,17β-diol,
11β-[4-(3-dimethylamino)propyl)phenyl]-estra-1,3,5(10)-triene-3,17β-diol
11β-[4-(1-piperidinyl)propyl)phenyl]-estra-1,3,5(10)-triene-3,17β-diol
4-chloro-11β-[4-[3-(1-pyrrolidinyl)propyl]phenyl]-estra-1,3,5(10)-triene-3,17β-diol
4-chloro-11β-[4-[3-(1-piperidinyl)propyl]phenyl]-estra-1,3,5(10)-triene-3,17β-diol
4-chloro-11β-[4-[3-(diethylamino)propyl]phenyl]-estra-1,3,5(10)-triene-3,17β-diol
17α-methyl-11β-[4-[3-(1-piperidinyl)propyl]phenyl]-estra-1,3,5(10)-triene-3,17β-diol,
4-chloro-17α-methyl-11β-[4-[3-(1-piperidinyl)propyl]phenyl]-estra-1,3,5(10)-triene-3,17β-diol,
11β-[4-[3-(1-piperidinyl)propyl]phenyl]-17α-(trifluoromethyl)-estra-1,3,5(10)-triene-3,17β-diol
(17R) 11β-[4-[3-(dimethylamino)propyl]phenyl]-spiro-(estra-1,3,5(10)-triene-17,2'(5'H)-furan)-3-ol
(17R) 4',5'-dihydro-11β-[4-[3-(dimethylamino)propyl] phenyl]-spiro-(estra-1,3,5(10)-triene-17,2'(3'H)-furan)-3-ol.

9. Process for the preparation of the compounds of formula (I) as defined in claim 1 in which a compound of formula (II) :

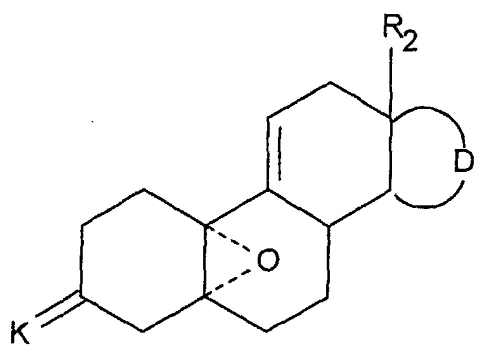

(II)

in which D and $R_2$ are as defined in claim 1, and K represents a protective group of the 3-keto function, is successively subjected to the following reactions:

a) the action of a compound of formula (III):

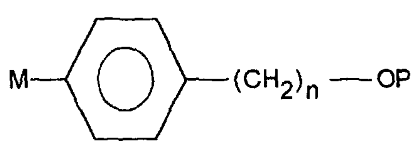

in which M represents a metallic derivative, P represents a protective group, of the alcohol, and n is an integer equal to 3, 4 or 5, then, if appropriate, deprotection of one or more protected reactive functions in order to obtain a compound of formula ($III_a$) :

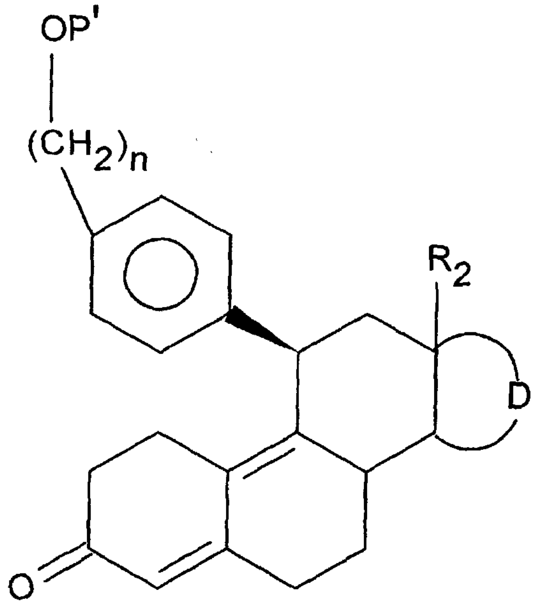

(IIIa)

P' having the same values as P as well as hydrogen,
b) the action, if appropriate, of a halogenation reagent in order to obtain a compound of formula ($III_b$):

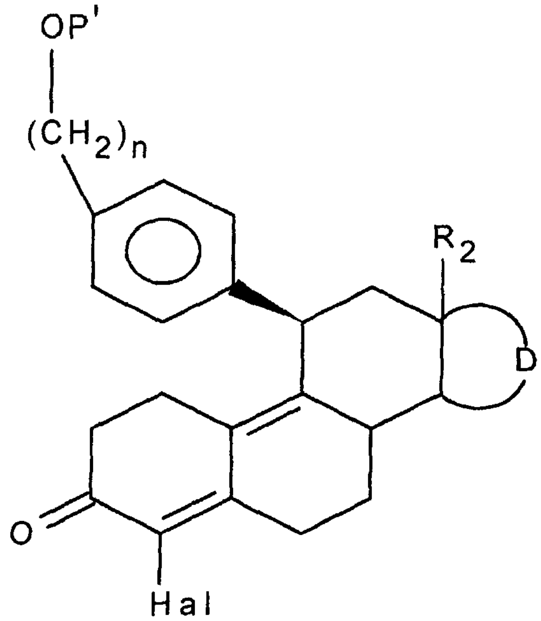

(IIIb)

Hal representing a halogen atom,
c) the action of an aromatization reagent of ring (A) on the compounds of formulae ($III_a$) and ($III_b$), after having, if appropriate, protected and/or activated the OH function, then the action of a base in order to obtain the compound of formula (IV):

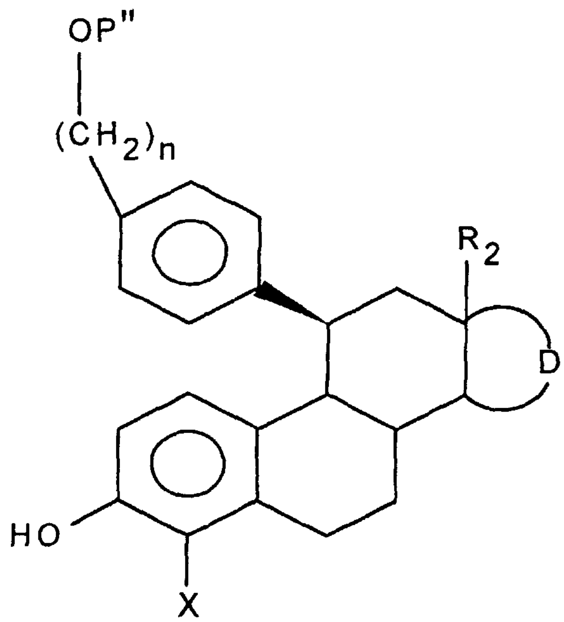

(IV)

P" having the same values as P' and being able to represent moreover an activating group, X being as defined in claim 1,
d) the action of an amine of formula (V):

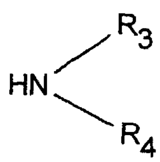

(V)

$R_3$ and $R_4$ being as defined in claim 1, this compound being . optionally in the form of a salt, in order to obtain certain compounds of formula (I), the compounds of formulae ($III_a$), ($III_b$), (IV) and (I) being subjected, if desired or if necessary, in an appropriate order, to one or more of the following reactions:

- protection/deprotection of the OH group or groups,
- alkylation/acylation of OH group or groups,
- the action of a reducing agent when D represents the remainder of a pentagonal ring as defined in claim 2 and $R_5$ and $R_6$ form together an oxo group,
- the action of an organometallic or of $CF_3SiMe_3$ on the compounds of formula (IV) or (I) with D representing the remainder of a pentagonal ring as defined in claim 2 and $R_5$ and $R_6$ form together an oxo group,
- the action of a lactonization agent on the compounds of formula (IV) or (I) with D representing the remainder of a pentagonal ring as defined in claim 2 and $R_5$ and $R_6$ form together an oxo group,
- the action of a reducing agent of the double bond, when D represents the remainder of a pentagonal ring as defined in claim 2, and $R_5$ and $R_6$ form together with the carbon which carries them an O-$(CH_2)_{l'}$ -CH=CH- group,
- the action of a reducing agent of the double bond, when D represents the remainder of a pentagonal ring as defined in claim 2, and $R_6$ is an alkenyl or alkynyl radical containing 2 to 6 carbon atoms,
- salification.

**10.** Process for the preparation according to claim 9 of the compounds of general formula (I') as defined in claim 3 in which a compound of formula (II'):

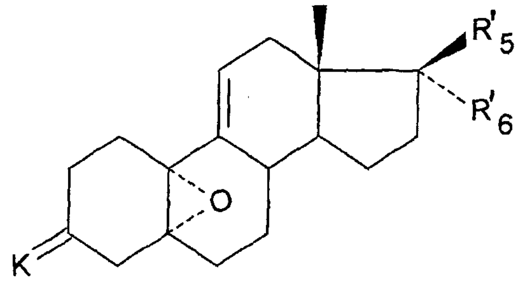

(II')

in which K, $R'_5$ and $R'_6$ are as defined in claim 3, or in which $R'_5$ is a CN radical and $R'_6$ is a protected hydroxyl, is successively subjected to the following reactions:

a) the action of a compound of formula (III'):

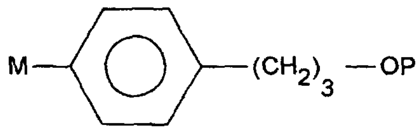

(III')

in which M and P are as defined previously, then deprotection of one or more protected reactive functions in order to obtain a compound of formula ($III'_a$):

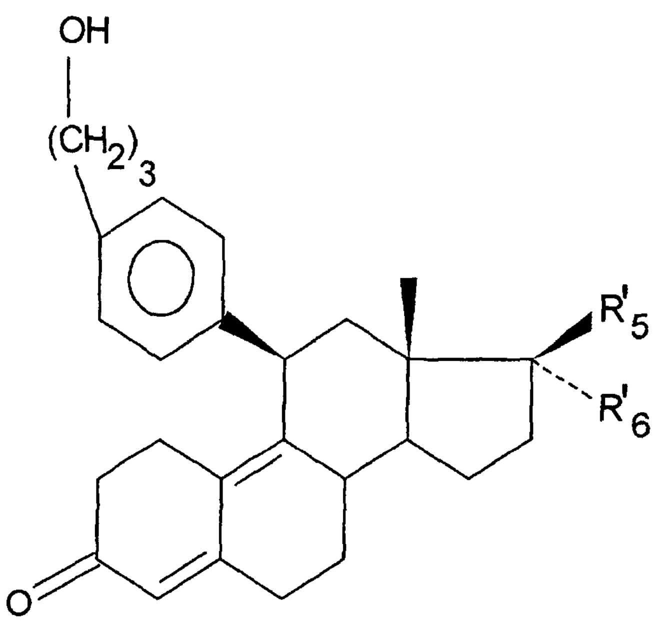

($III'_a$)

b) the action, if appropriate, of a halogenation reagent in order to obtain a compound of formula ($III'_b$):

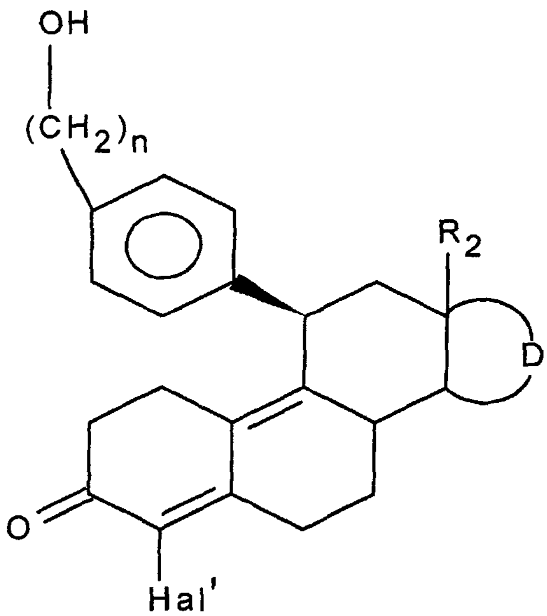

(III'b)

Hal' representing a chlorine or bromine atom,

c) the activation of the OH function then the action of an aromatization reagent of ring (A) on the compounds of formula ($III_a$) or ($III_b$), then the action of a base in order to obtain the compounds of formula (IV'):

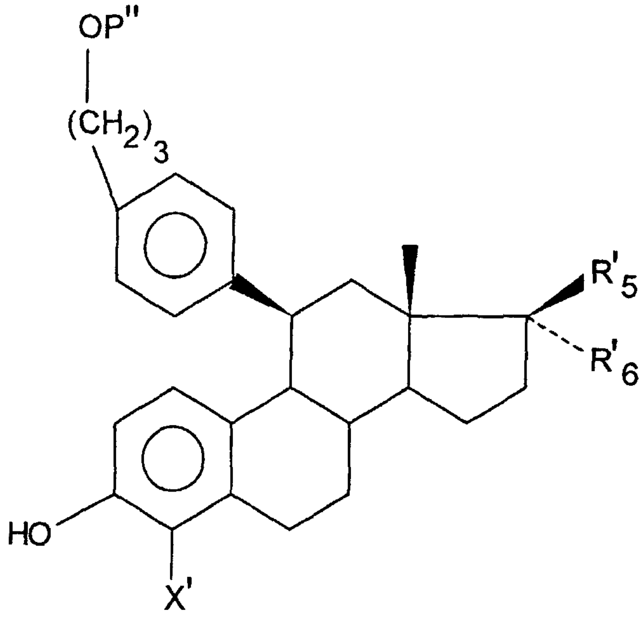

(IV')

X' and P" being as defined in claim 9,

d) the action of an amine of formula (V') :

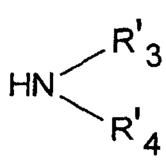

(V')

$R'_3$ and $R'_4$ being as defined previously, in order to obtain certain compounds of formula (I'), the compounds of formulae ($III'_a$), ($III'_b$), (IV') and (I') being subjected if desired or if necessary to one or more of the following reactions:

- protection/deprotection of the OH group or groups,
- alkylation/acylation of the OH group or groups,
- the action of a reducing agent when $R'_5$ and $R'_6$ form together an oxo group,
- the action of an organometallic or of $CF_3SiMe_3$ on the compounds of formula (IV') or (I') with $R'_5$ and $R'_6$ forming together an oxo group,
- the action of a lactonization agent on the compounds of formula (IV') or (I') with $R'_5$ and $R'_6$ forming together an oxo group,
- the action of a reducing agent of the double bond, when $R'_5$ and $R'_6$ form together with the carbon which carries them an O-$(CH_2)_{1'}$ -CH=CH- group,
- the action of a reducing agent of the double bond, when $R'_6$ is an alkenyl or alkynyl radical containing 2 to 6 carbon atoms,
- salification.

**11.** As medicaments, the compounds of formula (I) as defined in claim 1, as well as their addition salts with pharmaceutically acceptable acids or bases.

**12.** As medicaments, the compounds of formula (I) or (I') as defined in any one of claims 2 to 7, as well as their addition salts with pharmaceutically acceptable acids or bases.

**13.** As medicaments, the compounds as defined in claim 8, as well as their addition salts with pharmaceutically acceptable acids.

**14.** Pharmaceutical compositions containing one or more of the medicaments as defined in any one of claims 11, 12 or 13.

**15.** As new intermediate products, the compounds of general . formulae ($III_a$), ($III_b$), ($III'_a$), ($III'_b$), (IV) or (IV') as defined in claim 9 or 10.

**Patentansprüche**

**1.** Verbindungen der allgemeinen Formel (I):

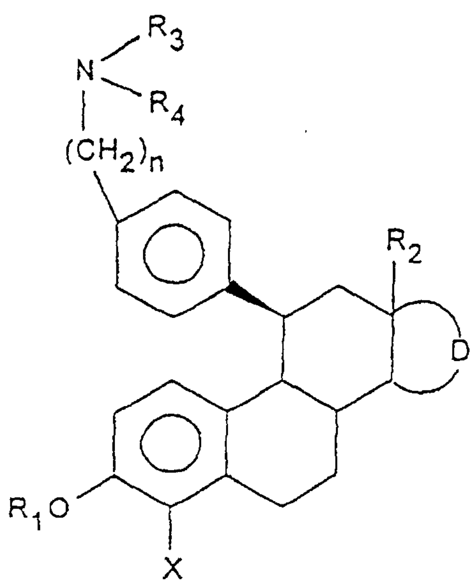

(I),

in der:

- $R_1$ ein Wasserstoffatom oder einen Rest $(CH_2)_m$-Ar, (CO)-Ar, $(CH_2)_m$ -Alk oder (CO)-Alk darstellt,
- $R_2$ einen von einem geradkettigen oder verzweigten, gesättigten oder ungesättigten Kohlenwasserstoff abgeleiteten Rest mit 1 bis 6 Kohlenstoffatomen darstellt,
- D den Rest eines gegebenenfalls substituierten und gegebenenfalls ungesättigten Fünf- oder Sechsringes darstellt,
- X ein Halogenatom oder ein Wasserstoffatom darstellt,
- n gleich 3, 4 oder 5 ist
- entweder $R_3$ und $R_4$, die gleich oder verschieden sein können, jeweils ein Wasserstoffatom oder eine Gruppe $(CH_2)_m$-Ar, $(CH_2)_m$-Het oder $(CH_2)_m$-Alk darstellen
  oder $R_3$ und $R_4$ zusammen mit dem Stickstoffatom, an das sie gebunden sind, einen gesättigten oder ungesättigten, aromatischen oder nicht aromatischen, mono- oder polyzyklischen Heterozyklus mit 3 bis 15 Kettengliedern bilden, der gegebenenfalls 1 bis 3 zusätzliche, unter Sauerstoff, Schwefel und Stickstoff ausgewählte Heteroatome aufweist und der substituiert oder nicht substituiert sein kann,

  wobei:

  Ar eine carbozyklische Arylgruppe mit 6 bis 18 Kohlenstoffatomen darstellt,
  Het einen aromatischen oder einen gesättigten oder nicht gesättigten nicht aromatischen Heterozyklus darstellt, der 1 bis 9 Kohlenstoffatome und 1 bis 5 unter dem Sauerstoffatom, dem Stickstoffatom und dem Schwefelatom ausgewählte Heteroatome aufweist,
  Alk einen von einem gesättigten oder ungesättigten, geradkettigen, verzweigten oder zyklischen nicht aromatischen Kohlenwasserstoff abgeleiteten Rest mit 1 bis 12 Kohlenstoffatomen darstellt,
  die Reste Ar, Het und Alk substituiert oder unsubstituiert sein können
  und
  m gleich 0, 1, 2 oder 3 ist,

sowie die Salze, die durch Addition dieser Verbindungen der Formel (I) an Basen oder Säuren gebildet werden.

**2.** Verbindungen der allgemeinen Formel (I) wie in Anspruch 1 definiert, wobei in dieser Formel (I) D den Rest eines Fünfringes der folgenden Formel darstellt:

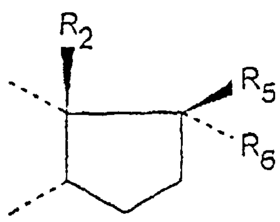

in der:

- $R_2$ dieselbe Bedeutung hat wie in Anspruch 1,
- entweder $R_5$ einen Rest OH, O-$(CH_2)_m$-Alk, O-(CO)-Alk, O-$(CH_2)_m$-Ar, O-(CO)-Ar, O-$(CH_2)_m$-Het oder O-(CO)-Het und $R_6$ ein Wasserstoffatom oder einen substituierten oder nicht substituierten Alkyl-, Alkenyl- oder Alkinylrest mit 1 bis 6 Kohlenstoffatomen darstellt, wobei m, Alk, Ar und Het wie in Anspruch 1 definiert zu verstehen sind,
  oder $R_5$ und $R_6$ zusammen mit dem Kohlenstoffatom, an das sie gebunden sind, einen der folgenden Zyklen bilden:

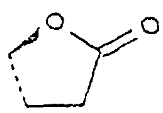 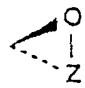 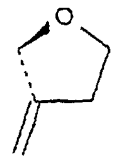

- wobei Z eine Gruppe $(CH_2)_1$ oder $CH=CH-(CH_2)_{1'}$ darstellt, in der 1 eine ganze Zahl von 1 bis 4 beziehungsweise 1' eine ganze Zahl von 1 bis 2 ist,

<u>oder</u> $R_5$ und $R_6$ gemeinsam eine Oxogruppe bilden,

sowie die Salze, die durch Addition dieser Verbindungen der Formel (I) an Säuren oder Basen gebildet werden.

**3.** Verbindungen der allgemeinen Formel (I) wie in Anspruch 1 oder 2 definiert, die der allgemeinen Formel (I') entsprechen:

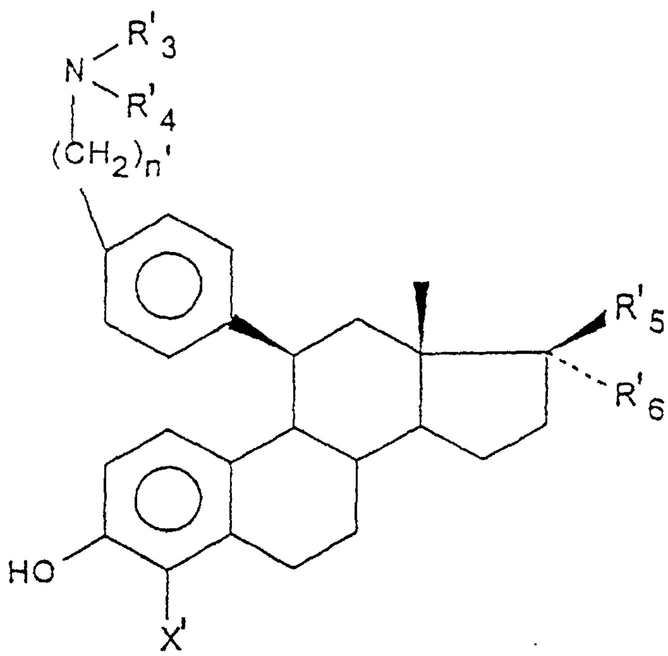

(I'),

in der:

- X' ein Chlor-, Brom- oder Wasserstoffatom darstellt,
- n' gleich 3 ist,
- <u>entweder</u> $R'_3$ und $R'_4$, die gleich oder verschieden sein können, jeweils einen Alkylrest mit 1 bis 6 Kohlenstoffatomen darstellen
  <u>oder</u> $R'_3$ und $R'_4$ zusammen mit dem Stickstoffatom, an das sie gebunden sind, einen gesättigten mono- oder polyzyklischen Rest mit 3 bis 15 Kettengliedern bilden, der gegebenenfalls ein zusätzliches, unter Sauerstoff, Schwefel und Stickstoff ausgewähltes Heteroatom aufweist,
  und
- $R'_5$ und $R'_6$ dieselbe Bedeutung haben wie $R_5$ beziehungsweise $R_6$ in Anspruch 2,

sowie die Salze, die durch Addition dieser Verbindungen der allgemeinen Formel (I') an Säuren oder Basen gebildet werden.

**4.** Verbindungen der allgemeinen Formel (I') wie in Anspruch 3 definiert,

wobei in dieser Formel (I'):

entweder $R'_5$ einen Rest OH und $R'_6$ ein Wasserstoffatom oder einen substituierten oder nicht substituierten Alkyl-, Alkenyl- oder Alkinylrest mit 1 bis 6 Kohlenstoffatomen darstellt
oder $R'_5$ und $R'_6$ zusammen mit dem Kohlenstoffatom, an das sie gebunden sind, einen der folgenden Zyklen bilden:

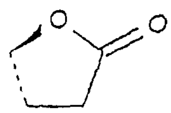
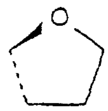
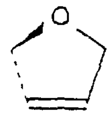
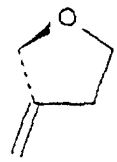

oder $R'_5$ und $R'_6$ zusammen eine Oxogruppe bilden,

sowie die Salze, die durch Addition dieser Verbindungen der allgemeinen Formel (I') an Säuren oder Basen gebildet werden.

**5.** Verbindungen der allgemeinen Formel (I') wie in Anspruch 3 oder 4 definiert, wobei in dieser Formel (I'):

- X' ein Chloratom oder ein Wasserstoffatom darstellt,
- entweder $R'_3$ und $R'_4$, die gleich oder verschieden sein können, jeweils einen Alkylrest mit 1 bis 6 Kohlenstoffatomen darstellen
  oder $R'_3$ und $R'_4$ zusammen mit dem Stickstoffatom, an das sie gebunden sind, einen der folgenden gesättigten Heterozyklen bilden:

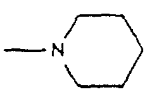
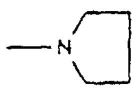
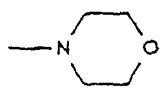
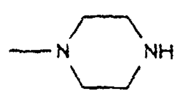
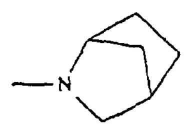

  und
- entweder $R'_5$ einen Rest OH und $R'_6$ ein Wasserstoffatom oder einen substituierten oder nicht substituierten Alkyl-, Alkenyl- oder Alkinylrest mit 1 bis 6 Kohlenstoffatomen darstellt,
  oder $R'_5$ und $R'_6$ zusammen mit dem Kohlenstoffatom, an das sie gebunden sind, einen der folgenden Zyklen bilden:

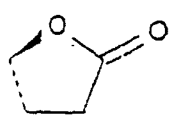
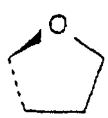
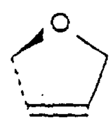
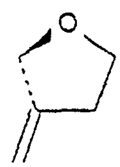

oder $R'_5$ und $R'_6$ zusammen eine Oxogruppe bilden,

sowie die Salze, die durch Addition dieser Verbindungen der allgemeinen Formel (I') an Säuren oder Basen gebildet werden.

**6.** Verbindungen der allgemeinen Formel (I) wie in einem der Ansprüche 1 bis 5 definiert, in der X ein Wasserstoffatom ist.

**7.** Verbindungen der allgemeinen Formel (I) wie in einem der Ansprüche 1 bis 5 definiert, in der X ein Chloratom ist.

**8.** Verbindungen der Formel (I) oder (I') wie in einem der Ansprüche 1 bis 7 definiert, die den folgenden Namen entsprechen:

3-Hydroxy-11β-[4-[3-(1-piperidinyl)propyl]phenyl]-estra-1,3,5(10)-trien-17-on;
3-Hydroxy-11β-[4-[3-(1-pyrrolidinyl)propyl]phenyl]-estra-1,3,5(10)-trien-17-on;
3-Hydroxy-11β-[4-[3-(dimethylamino)propyl]phenyl]-estra-1,3,5(10)-trien-17-on;
4-Chlor-3-hydroxy-11β-[4-[3-(1-piperidinyl)propyl]phenyl]-estra-1,3,5(10)-trien-17-on;
4-Chlor-3-hydroxy-11β-[4-[3-(1-pyrrolidinyl)propyl]phenyl]-estra-1,3,5(10)-trien-17-on;
4-Chlor-3-hydroxy-11β-[4-[3-(diethylamino)propyl]phenyl]-estra-1,3,5(10)-trien-17-on;
11β-[4-[3-(1-Pyrrolidinyl)propyl]phenyl]-estra-1,3,5(10)-trien-3,17β-diol;
11β-[4-[3-(Dimethylamino)propyl]phenyl]-estra-1,3,5(10)-trien-3,17β-diol;
11β-[4-[3-(1-Piperidinyl)propyl]phenyl]-estra-1,3,5(10)-trien-3,17β-diol;
4-Chlor-11β-[4-[3-(1-pyrrolidinyl)propyl]phenyl]-estra-1,3,5(10)-trien-3,17β-diol;
4-Chlor-11β-[4-[3-(1-piperidinyl)propyl]phenyl]-estra-1,3,5(10)-trien-3,17β-diol;
4-Chlor-11β-[4-[3-(diethylamino)propyl]phenyl]-estra-1,3,5(10)-trien-3,17β-diol;
17α-Methyl-11β-[4-[3-(1-piperidinyl)propyl]phenyl]-estra-1,3,5(10)-trien-3,17β-diol,
4-Chlor-17α-methyl-11β-[4-[3-(1-piperidinyl)propyl]phenyl]-estra-1,3,5(10)-trien-3,17β-diol;
11β-[4-[3-(1-Piperidinyl)propyl]phenyl]-17α-(trifluormethyl)-estra-1,3,5(10)-trien-3,17β-diol;
(17R) 11β-[4-[3-(Dimethylamino)propyl]phenyl]-spiro-(estra-1,3,5(10)-trien-17, 2'(5'H)-furan)-3-ol
und
(17R) 4',5'-Dihydro-11β-[4-[3-(dimethylamino)propyl]phenyl]-spiro-(estra-1,3,5(10)-trien-17, 2' (3'H)-furan)-3-ol.

**9.** Verfahren zur Herstellung von Verbindungen der Formel (I) wie in Anspruch 1 definiert, bei dem eine Verbindung der Formel (II):

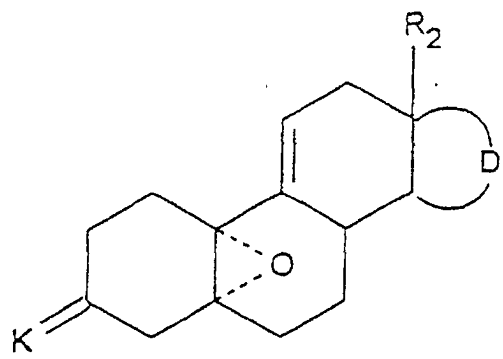

(II),

in der D und $R_2$ wie in Anspruch 1 definiert zu verstehen sind und K eine Schutzgruppe der 3-Keto-Funktion darstellt,
nacheinander folgenden Reaktionen unterzogen wird:

a) Umsetzung mit einer Verbindung der Formel (III):

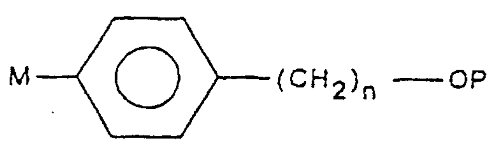

(III),

in der M ein Metallderivat, P eine Schutzgruppe der Alkoholfunktion und n eine ganze Zahl von 3, 4 oder 5 darstellt. und - sofern erforderlich - anschließende Entfernung einer oder mehrerer vorhandener Schutzgruppen von den geschützten reaktiven Funktionen, wobei eine Verbindung der Formel ($III_a$) erhalten wird:

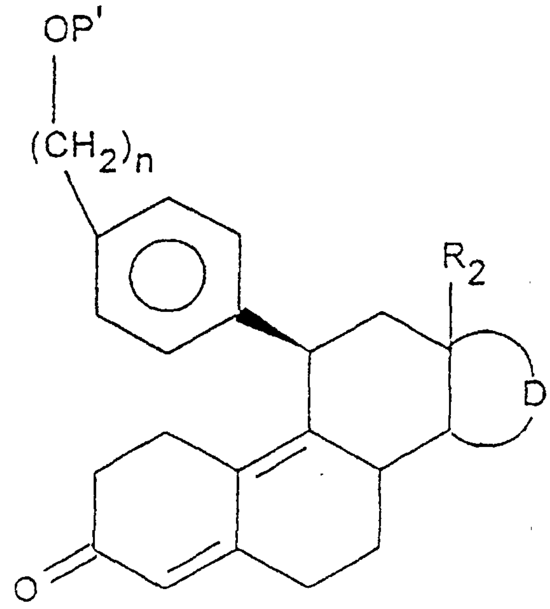

$(III_a)$,

in der P' Wasserstoff sein oder aber dieselbe Bedeutung wie P haben kann;

b) erforderlichenfalls Umsetzung mit einem Halogenierungsmittel, wobei eine Verbindung der Formel $(III_b)$ erhalten wird:

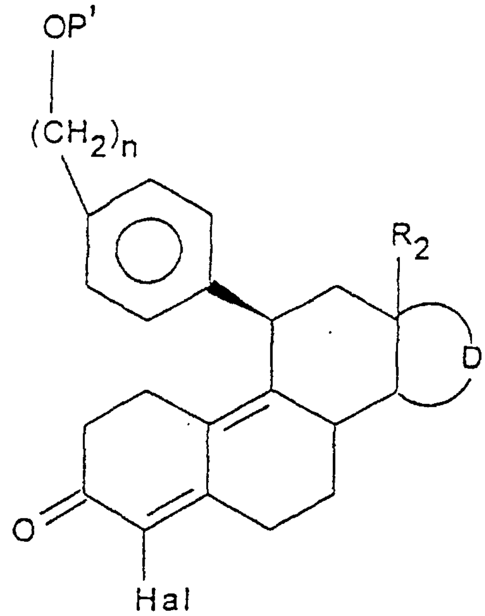

$(III_b)$,

in der Hal ein Halogenatom darstellt;

c) Umsetzung der Verbindungen der Formeln $(III_a)$ und $(III_b)$, deren OH-Funktion gegebenenfalls zuvor geschützt und/oder aktiviert wurde, mit einem Reagens, das die Aromatisierung des Ringes (A) bewirkt, sowie anschließende Umsetzung mit einer Base unter Erhalt einer Verbindung der Formel (IV):

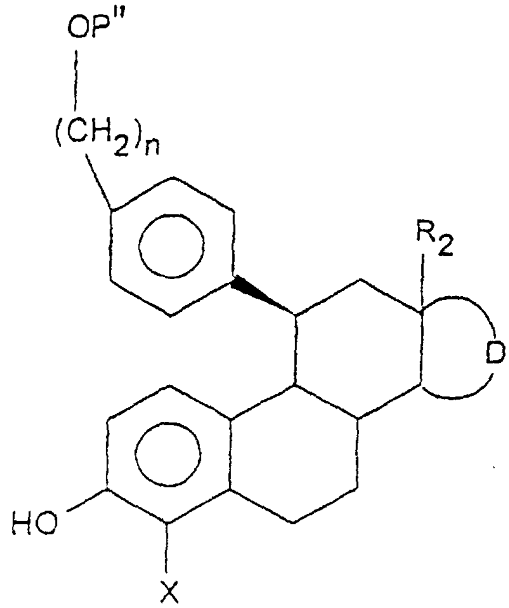

(IV),

in der P" dieselben Bedeutungen wie P' haben oder aber eine aktivierende Gruppe darstellen kann und in der X wie in Anspruch 1 definiert zu verstehen ist;

d) Umsetzung mit einem Amin der Formel (V):

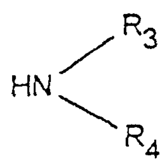

(V),

in der $R_3$ und $R_4$ wie in Anspruch 1 definiert zu verstehen sind,

wobei diese Verbindung (V) gebenenfalls in Form eines Salzes vorliegen kann,
unter Erhalt bestimmter Verbindungen der Formel (I),

wobei bei diesem Verfahren die Verbindungen der Formeln ($III_a$), ($III_b$), (IV) und (I)

- sofern gewünscht beziehungsweise erforderlich - in geeigneter Reihenfolge einer oder mehreren der folgenden Reaktionen unterzogen werden:
- Schutz der OH-Gruppe(n) durch Einbringen einer Schutzgruppe / Wiederherstellung der OH-Gruppe(n) durch Entfernen der Schutzgruppe,
- Alkylierung/Acylierung der OH-Gruppe(n),
- Umsetzung mit einem Reduktionsmittel, sofern D den Rest eines Fünfringes wie in Anspruch 2 definiert darstellt und $R_5$ und $R_6$ zusammen eine Oxogruppe bilden,
- Umsetzung der Verbindungen der Formel (IV) beziehungsweise (I), in denen D den Rest eines Fünfringes wie in Anspruch 2 definiert darstellt und $R_5$ und $R_6$ zusammen eine Oxogruppe bilden, mit einer Organometallverbindung oder mit $CF_3SiMe_3$,
- Umsetzung der Verbindungen der Formel (IV) beziehungsweise (I), in denen D den Rest eines Fünfringes wie in Anspruch 2 definiert darstellt und $R_5$ und $R_6$ zusammen eine Oxogruppe bilden, mit einem Reagens zur Lactonbildung,
- Umsetzung mit einem Reduktionsmittel für die Doppelbindung, sofern D den Rest eines Fünfringes wie in Anspruch 2 definiert darstellt und $R_5$ und $R_6$ zusammen mit dem Kohlenstoffatom, an das sie gebunden sind, eine Gruppe O - $(CH_2)_{l'}$ - CH=CH - bilden,
- Umsetzung mit einem Reduktionsmittel für die Doppelbindung, sofern D den Rest eines Fünfringes wie in

Anspruch 2 definiert darstellt und $R_6$ ein Alkenyl- oder Alkinylrest mit 2 bis 6 Kohlenstoffatomen ist, sowie

- Salzbildung.

**10.** Verfahren zur Herstellung von Verbindungen der allgemeinen Formel (I') wie in Anspruch 3 definiert nach Anspruch 9, bei dem eine Verbindung der Formel (II'):

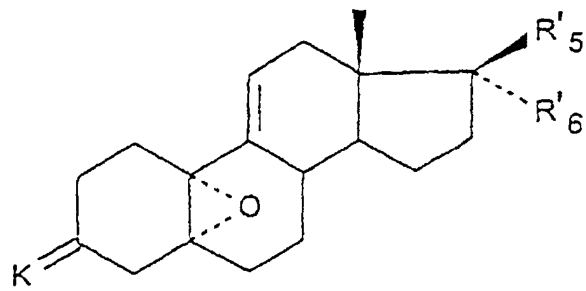

(II'),

in der K, $R'_5$ und $R'_6$ wie in Anspruch 3 definiert zu verstehen sind oder in der $R'_5$ ein Rest CN und $R'_6$ eine geschützte Hydroxylgruppe ist,
nacheinander folgenden Reaktionen unterzogen wird:

a) Umsetzung mit einer Verbindung der Formel (III'):

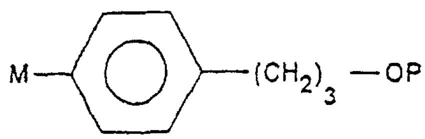

(III'),

in der M und P wie oben definiert zu verstehen sind,
und anschließende Entfernung einer oder mehrerer vorhandener Schutzgruppen von den geschützten reaktiven Funktionen, wobei eine Verbindung der Formel ($III'_a$) erhalten wird:

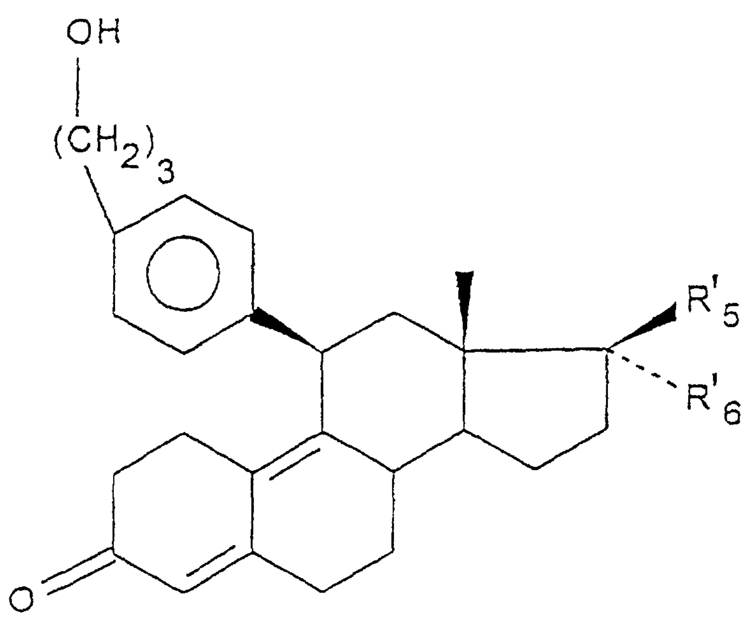

($III'_a$),

b) gegebenenfalls Umsetzung mit einem Halogenierungsmittel unter Erhalt einer Verbindung der Formel ($III'_b$):

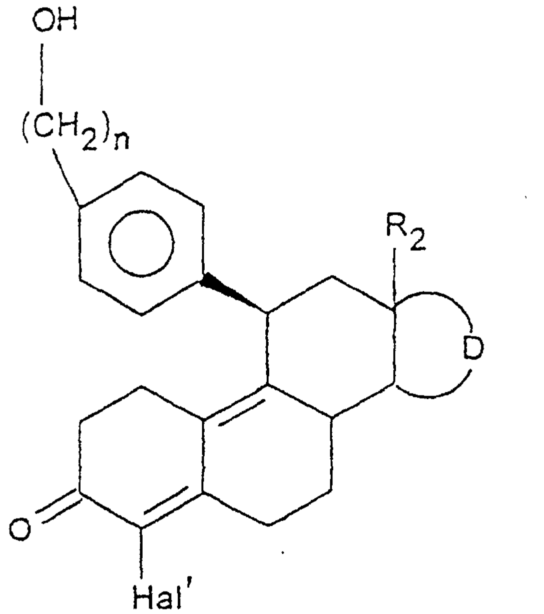

($III'_b$),

in der Hal' ein Chlor- oder Bromatom darstellt;

c) Aktivierung der OH-Funktion, anschließende Umsetzung der Verbindungen der Formel ($III_a$) beziehungsweise ($III_b$) mit einem Reagens, das die Aromatisierung des Ringes (A) bewirkt, sowie im Anschluss hieran Umsetzung mit einer Base unter Erhalt einer Verbindung der Formel (IV'):

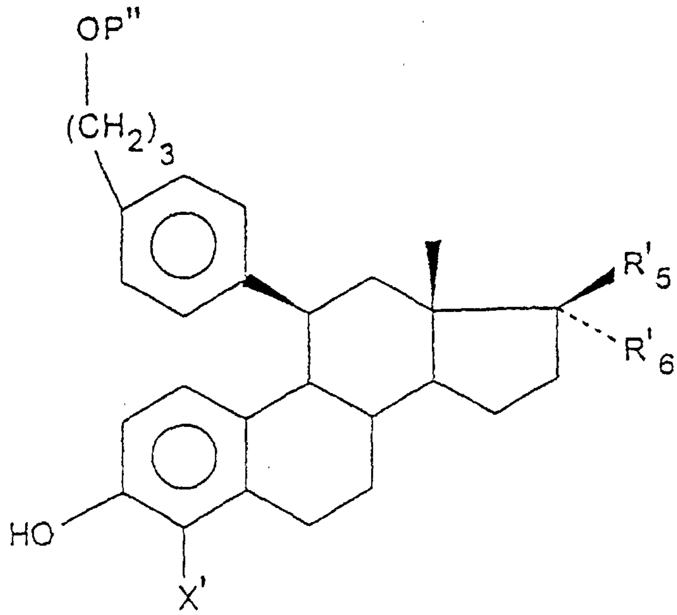

(IV'),

in der X' und P" wie in Anspruch 9 definiert zu verstehen sind;

d) Umsetzung mit einem Amin der Formel (V'):

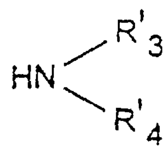

(V'),

in der $R'_3$ und $R'_4$ wie oben definiert zu verstehen sind,
unter Erhalt bestimmter Verbindungen der Formel (I'),

wobei bei diesem Verfahren die Verbindungen der Formeln ($III'_a$), ($III'_b$), (IV') und (I')

- sofern gewünscht beziehungsweise erforderlich - in geeigneter Reihenfolge einer oder mehreren der folgenden Reaktionen unterzogen werden:
- Schutz der OH-Gruppe(n) durch Einbringen einer Schutzgruppe / Wiederherstellung der OH-Gruppe(n) durch Entfernen der Schutzgruppe,
- Alkylierung/Acylierung der OH-Gruppe(n),
- Umsetzung mit einem Reduktionsmittel, sofern $R'_5$ und $R'_6$ zusammen eine Oxogruppe bilden,
- Umsetzung der Verbindungen der Formel (IV') beziehungsweise (I'), in denen $R'_5$ und $R'_6$ zusammen eine Oxogruppe bilden, mit einer Organometallverbindung oder mit $CF_3SiMe_3$,
- Umsetzung der Verbindungen der Formel (IV') beziehungsweise (I'), in denen $R'_5$ und $R'_6$ zusammen eine Oxogruppe bilden, mit einem Reagens zur Lactonbildung,
- Umsetzung mit einem Reduktionsmittel für die Doppelbindung, sofern $R'_5$ und $R'_6$ zusammen mit dem Kohlenstoffatom, an das sie gebunden sind, eine Gruppe O - $(CH_2)_{l'}$ - CH=CH - bilden,
- Umsetzung mit einem Reduktionsmittel für die Doppelbindung, sofern $R'_6$ ein Alkenyloder Alkinylrest mit 2 bis 6 Kohlenstoffatomen ist,
  sowie
- Salzbildung.

**11.** Verbindungen der Formel (I) wie in Anspruch 1 definiert sowie die Salze, die durch Addition dieser Verbindungen an pharmazeutisch akzeptable Säuren oder Basen gebildet werden, in ihrer Eigenschaft als Arzneimittel.

**12.** Verbindungen der Formel (I) oder (I') wie in einem der Ansprüche 2 bis 7 definiert sowie die Salze, die durch Addition dieser Verbindungen an pharmazeutisch akzeptable Säuren oder Basen gebildet werden, in ihrer Eigenschaft als Arzneimittel.

**13.** Verbindungen wie Anspruch 8 definiert sowie die Salze, die durch Addition dieser Verbindungen an pharmazeutisch akzeptable Säuren oder Basen gebildet werden, in ihrer Eigenschaft als Arzneimittel.

**14.** Pharmazeutische Zusammensetzungen, die ein oder mehrere Arzneimittel wie in einem der Ansprüche 11, 12 oder 13 definiert enthalten.

**15.** Verbindungen der allgemeinen Formeln ($III_a$), ($III_b$), ($III'_a$), ($III'_b$), (IV) und (IV') wie in Anspruch 9 oder 10 definiert in ihrer Eigenschaft als neue Zwischenprodukte.